# EUROPÄISCHE PATENTSCHRIFT

(11) **EP 1 474 099 B1**
(45) Veröffentlichungstag und Bekanntmachung des Hinweises auf die Patenterteilung: **09.09.2009**
(21) Anmeldenummer: 02785319.1
(22) Anmeldetag: 26.10.2002
(51) Int. Cl.: A61K 8/894, A61K 8/06, A61K 8/60, A61K 8/86, A61K 8/891, A61Q 17/02, A61Q 17/04, A61Q 19/00, A61Q 19/04

(54) **DÜNNFLÜSSIGE, SPRÜHBARE W/O EMULSIONEN**
LOW-VISCOSITY, SPRAYABLE W/O EMULSIONS
EMULSIONS E/H FLUIDES PULVERISABLES

(30) Priorität: 07.11.2001 DE 10154547
(43) Veröffentlichungstag der Anmeldung: 10.11.2004
(73) Patentinhaber: Beiersdorf AG, 20245 Hamburg (DE)
(72) Erfinder: GÖPPEL, Anja, 22527 Hamburg (DE); BLECKMANN, Andreas, 22926 Ahrensburg (DE)
(74) Vertreter: Wilke, Jochen
(86) Internationale Anmeldenummer: PCT/EP2002/011996
(87) Internationale Veröffentlichungsnummer: WO 2003/039508

(56) Entgegenhaltungen:
- WO-A-01/08648
- WO-A-02/39974
- WO-A-94/20067
- WO-A-96/14051
- WO-A1-98/15254
- DE-A- 19 844 261
- US-A- 5 143 722
- PATENT ABSTRACTS OF JAPAN vol. 2000, no. 06, 22. September 2000 (2000-09-22) & JP 2000 080017 A (KANEBO LTD), 21. März 2000 (2000-03-21)

## Beschreibung

Die vorliegende Erfindung betrifft dünnflüssige, sprühbare W/O Emulsionen mit einem reduzierten Silikongehalt für kosmetische und dermatologische Zubereitungen. In einer besonderen Ausführungsform betrifft die vorliegende Erfindung auch die Anwendung als Sonnenschutzprodukt.

Emulsionen gehören zu den dispersen Systemen. Emulsionen sind Zwei- oder Mehrphasensysteme von zwei oder mehr ineinander nicht oder nur wenig löslichen Flüssigkeiten. Die Flüssigkeiten (rein oder als Lösungen) liegen in einer Emulsion in einer mehr oder weniger feinen Verteilung vor, die im allgemeinen nur begrenzt stabil ist.

Nach dem heutigen Stand der Technik ist es schwer möglich, stabile, sprühbare W/O-Emulsionen mit einem niedrigen Silikonölgehalt herzustellen.
Derzeitige Formulierungen enthalten einen sehr hohen Gehalt an niedrig siedenden, sehr dünnflüssigen Silikonölen (z.B. 25-40 Gew.-% Cyclomethicone) zur Gewährleistung der Sprühbarkeit (Vergl. Patent US 4,563,346). Diese Silikonöle bilden dann - fast ausschließlich - die kontinuierliche Phase der Emulsion.
Des Weiteren werden zur Stabilisierung dünnflüssiger W/O-Emulsionen Schichtsilikate (WO 9614051) oder Organopolysiloxane (WO 9618374) eingesetzt. Dadurch ist es mit herkömmlichen Pumpsystemen nicht möglich, ein feines und gleichmäßiges Sprühbild zu erzeugen.

Ein weiterer Nachteil des Standes der Technik ist es, dass sich in derartigen Emulsionen (mit hohem Silikonölgehalt) bei Raumtemperatur feste UV-Filter aus der Gruppe der Triazine nur sehr begrenzt lösen und sich daher nur Sonnenschutzprodukte mit geringem Lichtschutz-faktor (LSF, SPF) herstellen lassen.

WO-A-0108648 beschreibt scheinbar sprühbare W/O-Emulsionen mit Silikonemulgatoren und Emulgatoren mit HLB Werten kleiner 7 bzw. größer 10.
WO-A-9815254 beschreibt gelförmige W/O-Emulsionen auf Mikroemulsionbasis umfassend verschiedenen W/O Emulgatoren in Kombination mit Silikonverdickern bzw. Vernetzern.

Aufgabe der vorliegenden Aufgabe war es daher, den Stand der Technik zu bereichern und seinen Nachteilen abzuhelfen.

Es war überraschend und für den Fachmann nicht vorauszusehen, dass sich dünnflüssige sprühbare W/O Emulsionen mit einem reduzierten Silikongehalt als kosmetische und dermatologische Zubereitungen herstellen lassen, enthaltend - neben weiteren kosmetischen/dermatologischen Zusatz- bzw. Hilfsstoffen - ein Emulgatorsystem aus:
A mindestens einem Silikonemulgator (W/S) mit einem HLB-Wert ≤ 8,
B mindestens einem W/O-Emulgator mit einem HLB-Wert < 7 und
C mindestens einem O/W-Emulgator mit einem HLB-Wert > 10,
und einem Silikonölgehalt unter 25 Gew.-% (bezogen auf das Gesamtgewicht der Zubereitung) und einer oder mehreren Lipide und/oder Öle enthaltenden Fettphase, wobei der oder die W/O-Emulgator(en) B aus der Klasse der Polyglycerin-emulgatoren gewählt wird/werden.

Die erfindungsgemäßen Zubereitungen stellen in jeglicher Hinsicht überaus befriedigende Präparate dar. Es war insbesondere überraschend, dass die aus den erfindungsgemäßen Zubereitungen hergestellten Emulsionen eine hohe Löslichkeit für UV-Filter aus der Gruppe der Triazine aufweisen. Des Weiteren lassen sich auch Repellentien und auch Selbstbräunungssubstanzen (z.B. Dihydroxyacteton) stabil in diese neuartigen Emulsionen einarbeiten.

Dementsprechend eignen sich Zubereitungen im Sinne der vorliegenden Erfindung ganz besonders, um als Grundlage für Produktformen mit vielfältigen Anwendungszwecken zu dienen.

Erfindungsgemäß können die Silikonemulgatoren A vorteilhaft aus der Gruppe der Alkylmethiconcopolyole und/oder Alkyl-Dimethiconcopolyole gewählt werden, insbesondere aus der Gruppe der Verbindungen, welche gekennzeichnet sind durch die folgende chemische Struktur: 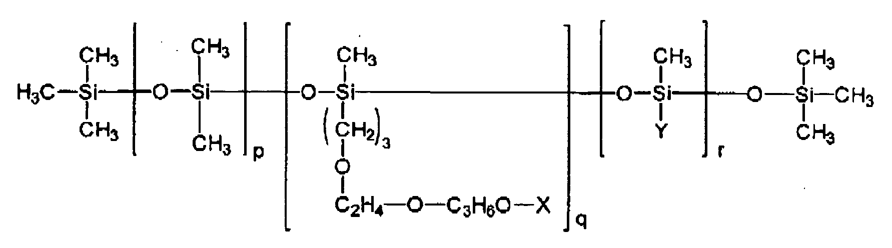 bei welcher X und Y unabhängig voneinander gewählt werden aus der Gruppe H (Wasserstoff) sowie der verzweigten und unverzweigten Alkylgruppen, Acylgruppen und Alkaxygruppen mit 1 - 24 Kohlenstoffatomen, p eine Zahl von 0 - 200 darstellt, q eine Zahl von 1-40 darstellt, und r eine Zahl von 1-100 darstellt.

Ein Beispiel für besonders vorteilhaft im Sinne der vorliegenden Erfindung zu verwendende Silikonemulgatoren sind Dimethiconcopolyole, welche von der Gesellschaft Th. Goldschmidt AG unter den Warenbezeichnungen ABIL® B 8842, ABIL® B 8843, ABIL® B 8847, ABIL® B 8851, ABIL® B 8852, ABIL® B 8863, ABIL® B 8873 und ABIL® B 88183 verkauft werden.

Ein weiteres Beispiel für besonders vorteilhaft im Sinne der vorliegenden Erfindung zu verwendende grenzflächenaktiven Substanzen ist das Cetyl Dimethiconcopolyol, welches von der Gesellschaft Th. Goldschmidt AG unter der Warenbezeichnung ABIL® EM 90 verkauft wird.

Ein weiteres Beispiel für besonders vorteilhaft im Sinne der vorliegenden Erfindung zu verwendende grenzflächenaktiven Substanzen ist das Cyclomethicon Dimethiconcopolyol, welches von der Gesellschaft Th. Goldschmidt AG unter der Warenbezeichnung ABIL® EM 97 verkauft wird.

Weiterhin hat sich als ganz besonders vorteilhaft der Emulgator Laurylmethiconcopolyol herausgestellt, welcher unter der Warenbezeichnung Dow Coming® 5200 Formulation Aid von der Gesellschaft Dow Coming Ltd. erhältlich ist.

Ein weiterer vorteilhafter Silikonemulgator ist ,Octyl Dimethicon Ethoxy Glucosid' der Firma Wacker.

Die Gesamtmenge an erfindungsgemäß verwendeten Silikonemulgatoren A in den erfindungsgemäßen kosmetischen oder dermatologischen Zubereitungen wird vorteilhaft aus dem Bereich von 0,1 - 10,0 Gew.-%, bevorzugt 0,5 - 5,0 Gew.-% gewählt, bezogen auf das Gesamtgewicht der Zubereitungen.

Der oder die W/O-Emulgatoren B werden erfindungsgemäß vorzugsweise gewählt aus der folgenden Gruppe:

Polyglyceryl-3-Oleat, Polyglyceryl-3 Diisostearat, Petrolatum im Gemisch mit Ozokerit und hydriertem Ricinusöl und Glycerylisostearat und Polyglyceryl-3-oleat, Polyglyceryl-4-isostearat, Polyglyceryl-4-isostearat, Polyglyceryl-2-dipolyhydroxystearat, Polyglyceryl-3-Diisostearat, Polyglyceryl-4-dipolyhydroxystearat, Diisostearoylpolyglyceryl-3-diisostearat, Polyglyceryl-2-dipolyhydroxystearat, Polyglyceryl-3-dipolyhydroxystearat, Polyglyceryl-4-dipolyhydroxystearat, Polyglyceryl-3-dioleat.

Der oder die O/W-Emulgatoren C werden erfindungsgemäß vorzugsweise aus der folgenden Gruppe gewählt:

Glycerylstearat im Gemisch mit Ceteareth-20, Ceteareth-25, Ceteareth-6 im Gemisch mit Stearylalkohol, Cetylstearylalkohol im Gemisch mit PEG-40-Ricinusöl und Natriumcetylstearylsulfat, Triceteareth-4 Phosphat, Glycerylstearat, Natriumcetylstearylsulfat, Lecithin Trilaureth-4 Phosphat, Laureth-4 Phosphat, Stearinsäure, Propylenglycolstearat SE, PEG-25-hydriertes Ricinusöl, PEG-54-hydriertes Ricinusöl, PEG-6 Caprylsäure/Caprinsäureglyceride, Glyceryloleat im Gemisch mit Propylenglycol, PEG-9-Stearat, PEG-20 Stearat, PEG-30-Stearat, PEG-40-Stearat, PEG-100-Stearat, Ceteth-2, Ceteth-20, Polysorbate-20, Polysorbate-60, Polysorbate-65, Polysorbate-100, Glycerylstearat im Gemisch mit PEG-100 Stearat, Glycerylmyristat, Glyceryllaurat, PEG-40-Sorbitanperoleat, Laureth-4, Ceteareth-3, Isostearylglycerylether, Cetylstearylalkohol im Gemisch mit Natrium Cetylstearylsulfat, Laureth-23, Steareth-2, Glycerylstearat im Gemisch mit PEG-30 Stearat, PEG-40-Stearat, Glycol. Distearat, PEG-22-Dodecyl Glycol Copolymer, Polyglyceryl-2-PEG-4-Stearat, Ceteareth-12, Ceteareth-20, Ceteareth-30, Methylglucosesesquistearat, Steareth-10, PEG-20-Stearat, Steareth-2 im Gemisch mit PEG-8 Distearat, Steareth-21, Steareth-20, Isosteareth-20, PEG-45/ Dodecylglycol-Copolymer, Methoxy-PEG-22/Dodecylglycol-Copolymer, PEG-40-Sorbitanperoleat, PEG-40-Sorbitanperisostearat, PEG-20-Glycerylstearat, PEG-20-Glycerylstearat, PEG-8-Bienenwachs, Polyglyceryl-2-laurat, Isostearyldiglycerylsuccinat, Stearamidopropyl-PGdimoniumchloridphosphat, Glycerylstearat SE, Ceteth-20, Triethytcitrat, PEG-20-Methylglucosesesquistearat, Glycerylstearatcitrat, Cetylphosphat, Cetearyl Sulfat, Sorbitansesquioleat, Triceteareth-4-Phosphat, Trilaureth-4-Phosphat, Polyglycerylmethylglucosedistearat, Kaliumcetylphosphat, Isosteareth-10, Polyglyceryl-2-sesquiisostearat, Ceteth-10, Oleth-20, Isoceteth-20, Glycerylstearat im Gemisch mit Ceteareth-20, Ceteareth-12, Cetylstearylalkohol und Cetylpalmitat, Cetylstearylalkohol im Gemisch mit PEG-20 Stearat, PEG-30-Stearat, PEG-40-Stearat, PEG-100-Stearat.

Es ist erfindungsgemäß vorteilhaft, die Gewichtsverhältnisse von Emulgator A zu Emulgator B zu Coemulgator C (A : B : C) wie a : b : c zu wählen, wobei a, b und c unabhängig voneinander rationale Zahlen von 1 bis 5, bevorzugt von 1 bis 3 darstellen können. Insbesondere bevorzugt ist ein Gewichtsverhältnis von etwa 2 : 1 : 1

Es ist vorteilhaft im Sinne der vorliegenden Erfindung, die Gesamtmenge der Emulgatoren A, B und C aus dem Bereich von 0,1 bis 15 Gew.-%, vorteilhaft von 0,5 bis 10 Gew.-%, insbesondere von 2 bis 10 Gew.-%, jeweils bezogen auf das Gesamtgewicht der Formulierung, zu wählen.

Es wird bevorzugt, die Ölphase der erfindungsgemäßen Zubereitungen zu mindestens 2,0 Gew.-%, bezogen auf das Gesamtgewicht der Zubereitungen, aus der Gruppe der cyclischen und/oder linearen Silicone zu wählen, welche im Rahmen der vorliegenden Offenbarung auch als "Siliconöle" bezeichnet werden. Solche Silicone oder Siliconöle können als Monomere vorliegen, welche in der Regel durch Strukturelemente charakterisiert sind, wie folgt:

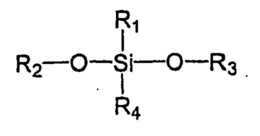

Erfindungsgemäß vorteilhaft einzusetzende lineare Silicone mit mehreren Siloxyleinheiten werden im allgemeinen durch folgendes Strukturelement charakterisiert: 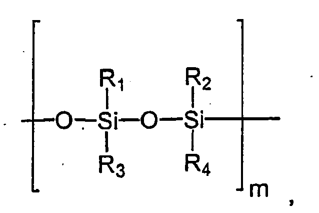 wobei die Substituenten an den Siliciumatome R₁ bis R₄ die gleichen oder unterschiedliche Alkylreste und/oder Arylreste sein können. Die Kettenlänge m kann dabei Werte von 2 - 200.000 annehmen.

Erfindungsgemäß vorteilhaft einzusetzende cyclische Silicone werden im allgemeinen durch folgendes Strukturelement charakterisiert 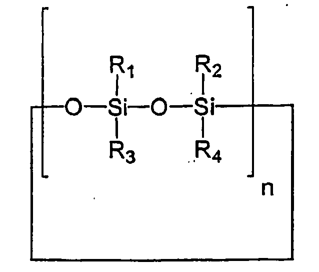 wobei die Substituenten an den Siliciumatome R₁ bis R₄ die gleichen oder unterschiedliche Alkylreste und/oder Arylreste sein können. Die Ringgröße n kann dabei Werte von 3/2 bis 20 annehmen. Gebrochene Werte für n berücksichtigen, daß eine ungeradzahlige Anzahl von Siloxylgruppen im Cyclus vorhanden sein kann.

Vorteilhaft wird Phenyltrimethicon als Siliconöl gewählt. Auch andere Silikonöle, wie beispielsweise Dimethicon, Phenyldimethicon, Cyclomethicone (beispielsweise Hexamethylcyclotrisiloxan, Octamethylcyclotetrasiloxan, Cyclopentasiloxan, Cyclohexasiloxan sowie Mischungen aus diesen Komponenten), Polydimethylsiloxan, Poly(methylphenylsiloxan), Cetyldimethicon, Behenoxydimethicon sind vorteilhaft im Sinne der vorliegenden Erfindung zu verwenden. Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle von ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Polysiloxan-polyalkyl-polyether-copolymere wie das Cetyl-Dimethicon-Copolyol, das (Cetyl-Dimethicon-Copolyol (und) Polyglyceryl-4-Isostearat (und) Hexyllaurat)

Vorteilhaft wird Cyclomethicon als erfindungsgemäß zu verwendendes Silikonöl eingesetzt. Aber auch andere Silikonöle sind vorteilhaft im. Sinne der vorliegenden Erfindung zu verwenden, beispielsweise Dimethicon (Polydimethylsiloxan) sowie Phenyltrimethicon bzw. Kombinationen aus den hier genannten Substanzen.

Es ist vorteilhaft im Sinne der vorliegenden Erfindung, die Gesamtmenge der Silikonöle auf 2 bis 25 Gew.-% zu beschränken. Erfindungsgemäß ist insbesondere eine Gesamtmenge der Silikonöle von 5 bis 20 Gew.-% und ganz besonders eine Gesamtmenge von 10 bis 15 Gew.-% - immer bezogen auf die Gesamtmenge - vorteilhaft.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Die Ölphase der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkemöl, Distelöl, Nachtkerzenöl, Macadamianußöl und dergleichen mehr.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft, gewählt werden aus der Gruppe Octylpalmitat, Octylcocoat, Octylisostearat, Octyldodeceylmyristat, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetisch, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung Cetiol CC bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum)_{;} Paraffinöl, Squalan und Squalen, Polyolefine, hydrogenierte Polyisobutene und Isohexadecan. Unter den Polyolefinen sind Polydecene die bevorzugten Substanzen.

Das oder die Lipide werden erfindungsgemäß aus Gruppe der natürlichen und/oder synthetischen Lipide gewählt. Vorzugsweise verwendet man: C12-C15 Alkylbenzoat, Capric/Caprylic Triglycerid, Butylen Glycol Dicaprylat/Dicaprat, Octyldodekanol, Dicaprylyl Carbonat, Dicaprylyl Ether, Mineralöl.

Besonders vorteilhaft sind ferner Mischungen aus Cyclomethicon, Dicaprylylcarbonat und C₁₂₋₁₅-Alkybenzoat aus Cyclomethicon, Dimethicon, Butylenglycol Dicaprylat/Dicaprat, Dicaprylyl Carbonat und Mineralöl.

Vorteilhaft beträgt der Gehalt an der Fettphase zwischen.1 und 80 Gew.-%, bezogen auf das Gesamtgewicht der Zubereitungen, bevorzugt 2,5 - 70 Gew.-%, insbesondere bevorzugt 5 - 60 Gew.-%.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Butylen Glykol, Ethylenglykol, Ethylhexylglycerin, Ethylenglykolmonoethyl- oder -monobutylether, Propylenglykolmonomethyl, -monoethyl- oder -monobutylether, Diethylenglykolmonomethyl- oder - monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Vorteilhafte Konservierungsmittel im Sinne der vorliegenden Erfindung sind beispielsweise Formaldehydabspalter (wie z. B. DMDM Hydantoin [z.B. Glydant®]), lodopropylbutylcarbamat (z. B. unter den Handelsbezeichnungen Koncyl-L, Koncyl-S und Konkaben LMB von der Fa. Lonza erhältlich), Parabene, Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Ethylhexyloxyglycerin, Glycine Soja etc.

Des Weitern können Feuchthaltemittel bzw. sogenannte Moisturizer enthalten sein. Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9).

Die erfindungsgemäßen Zusammensetzungen können ferner gegebenenfalls in der Kosmetik übliche Zusatzstoffe, beispielsweise Parfüm, Verdicker, Desodorantien, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate enthalten.

Die erfindungsgemäßen kosmetischen und dermatologischen Zubereitungen können Farbstoffe und/oder Farbpigmente enthalten, insbesondere wenn sie in Form von dekorativen Kosmetika vorliegen. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch.
Vorteilhafte Farbpigmente sind beispielsweise Titandioxid, Glimmer, Eisenoxide (z. B. Fe₂O₃, Fe₃O₄, FeO(OH)) und/oder Zinnoxid.
Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe Colour Index, 3. Auflage, Society of Dyers and Colourists, Bradford, England, 1971 entnommen.

| **Chemische oder sonstige Bezeichnung** | **CIN** | **Farbe** |
|---|---|---|
| Pigment Green | 10006 | grün |
| Acid Green 1 | 10020 | grün |
| 2,4-Dinitrohydroxynaphthalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-benzol-5-sulfosäure | 13015 | gelb |
| 2,4-Dihydroxy-azobenzol-4'-sulfosäure | 14270 | orange |
| 2-(2,4-Dimethylphenylazo-5-sulfosäure)-1-hydroxynaphthalin-4-sulfosäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sülfophenylazo)-2-hydroxynaphthalin | 15510 | orange |
| 1-(2-Sulfosäure-4-chlor-5-carbonsäure-1-phenylazo)-2-hydroxynaphthalin | 15525 | rot |
| 1-(3-Methyl-phenylazo-,4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4.-phenylazo-2-naphthylcarbonsäure | 15800 | rot |
| 1-(2-Sulfo-4-methyl-1-phenylazo)-2-naphthylcarbonsäure | 15850 | rot |
| 1-(2-Sulfo-4-methyl-5-chlor-1-phenylazo)=2-hydroxy-naphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1 -naphthylazo)-2-naphthol-6,8-disulfosäure | 16255 | rot |
| 1 -(4-Sulfo-1-naphthylazo)-2-naphthol-3,6,8-trisulfosäure | 16290 | rot |
| 8-Amino-2 -phenylazo- 1 -naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Suffo-1-phenylazo)-1-(4-sulfophenyl)-5-hydroxy-pyrazolon-3-carbonsäure | 19140 | gelb |
| Pigment Yellow 16 | 20040 | gelb |
| 2,6-(4'-Sulfo-2", 4"-dimethyl)-bis-phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"-Sulfo-1"-phenylazo)-7'-sulfo-1'-naphthylazo]-1-hydroxy-7-aminonaphthalin-3,6-disulfosäure | 27755 | schwarz |
| 4'-[(4"-Sulfo-1"-phenylazo)-7'-sulfo-1'-naphthylazo]-1-hydroXy⁻8-acetyl-aminonaphthalin-3,5-disulfosäure | 28440 | schwarz |
| Direct Orange 34, 39, 44, 46, 60 | 40215 | orange |
| Food Yellow | 40800 | orange |
| trans-β-Apo-8'-Carotinaldehyd (C₃₀) | 40820 | orange |
| trans-Apo-8'-Carotinsäure (C₃₀)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenyl-carbinol | 42051 | blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino)-phenyl-(2-sulfophenyl)-methylen-(N- | 42090 | blau |
| ethyl-N-p-sulfo-benzyl)Δ^{2,5}-cyclohexadienimin | | |
| Acid Green 9 | 42100 | grün |
| Diethyl-di-sulfobenzyl-di-4-amino-2-chlor-di-2-methyl-fuchsonimmonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethylN-m-sulfobenzyl-fuchsonimmonium | 42735 | blau |
| 4'-(N-Dimethyl)-amino-4"-(N-phenyl)-aminonaphtho-N-dimethylfuchsonimmonium | 44045 | blau |
| 2-Hydroxy-3,6-disulfo-4,4'-bis-dimethylaminonaphthofuchsonimmo- nium | 44090 | grün |
| Acid Red 52 | 45100 | rot |
| 3-(2'-Methylphenylamino)-6-(2'-methyl-4'-sulfophenylamino)-9-(2°-carboxyphenyl)-xantheniumsalz | 45190 | violett |
| Acid Red 50 . | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid Violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4.-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidino)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,T-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha-, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiße |
| calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Mangananimoniumdiphosphat | 77742 | violett |
| Manganphosphat; Mn₃(PO₄)₂ · 7 H20 | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalze, Anthocyane | | rot |
| Aluminium-, Zink-, Magnesium- und Calciumstearat | | weiß |
| Bromthymolblau | | blau |
| Bromkresolgrün | | grün |
| Acid Red 195 | | rot |

Sofern die erfindungsgemäßen Formulierungen in Form von Produkten vorliegen, welche im Gesicht angewendet werden, ist es günstig, als Farbstoff eine oder mehrere Substanzen aus der folgenden Gruppe zu wählen: 2,4-Dihydroxyazobenzol, 1-(2'-Chlor-4'-nitro-1'-phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthyl-azo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Ferner vorteilhaft sind öllösliche Naturfarbstoffe, wie z. B. Paprikaextrakte, β-Carotin oder Cochenille.

Vorteilhaft im Sinne der vorliegenden Erfindung sind ferner Formulierungen mit einem Gehalt an Perlglanzpigmenten. Bevorzugt sind insbesondere die im folgenden aufgelisteten Arten von Perlglanzpigmenten:
1. Natürliche Perlglanzpigmente, wie z. B.
   ■ "Fischsilber" (Guanin/Hypoxanthin-Mischkristalle aus Fischschuppen) und
   ■ "Perlmutt" (vermahlene Muschelschalen)
2. Monokristalline Perlglanzpigmente wie z. B. Bismuthoxychlorid (BiOCl)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteilhaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| **Gruppe** | **Belegung / Schichtdicke** | **Farbe** |
|---|---|---|
| **Silberweiße Perglanzpigmente** | TiO₂: 40 - 60 nm | silber |
| **Interferenzpigmente** | TiO₂: 60-80nm | gelb |
| | TiO₂: 80 - 100 nm | rot |
| | TiO₂: 100-140 nm | blau |
| | TiO₂: 120-160 nm | grün |
| **Farbglanzpigmente** | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| **Kombinationspigmente** | TiO₂ / Fe₂O₃ | Goldtöne |
| | TiO₂ / Cr₂O₃ | grün |
| | TiO₂ / Berliner Blau | tiefblau |
| | TiO₂ / Carmin | rot |

Besonders bevorzugt sind z.B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden. Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO₂ und Fe₂O₃ beschichtete SiO₂-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Eisenperlglanzpigmente, welche ohne die Verwendung von Glimmer hergestellt werden. Solche Pigmente sind z. B. unter dem Handelsnamen Sicopearl Kupfer 1000 bei der Firma BASF erhältlich.

Besonders vorteilhaft sind ferner auch Effektpigmente, welche unter der Handelsbezeichnung Metasomes Standard / Glitter in verschiedenen Farben (yello, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

### Wirkstoffe

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Carnosin und deren Derivate (z. B. Anserin), Carotinoide, Carotine (z. B. α-Carotin, β-carotin, Lycopin) und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ -Linoleyl-, Cholesteryl - und Glycerylester) sowie deren Salze, Dilaurylthiodipropionat, Distearylthiodipropionat, Thiodipröpionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg - Ascorbylphosphat, Ascorbylacetat), Tocopherole und Derivate (z. B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, ZnSO₄) Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Eine erstaunliche Eigenschaft der erfindungsgemäßen Zubereitungen ist, daß diese sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Erfindungsgemäß können die Wirkstoffe (eine oder mehrere Verbindungen) auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z. B. Hydrocortison-17-valerat, Vitamine der B- und D-Reihe, sehr günstig das Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die gamma-Linolensäure, Ölsäure, Eicosapentaensäure, Docosahexaensäure und deren Derivate, Chloramphenicol, Coffein, Prostaglandine, Thymol, Campher, Extrakte oder andere Produkte pflanzlicher und tierischer Herkunft, z. B. Nachtkerzenöl, Borretschöl oder Johannisbeerkernöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl, Eucerit^{®} und Neocerit^{®}.

Besonders vorteilhaft werden der oder die Wirkstoffe ferner gewählt aus der Gruppe der NO-Synthasehemmer, insbesondere wenn die erfindungsgemäßen Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut dienen sollen.

Bevorzugter NO-Synthasehemmer ist das Nitroarginin.

Weiter vorteilhaft werden der oder die Wirkstoffe gewählt aus der Gruppe, welche Catechine und Gallensäureester von Catechinen und wäßrige bzw. organische Extrakte aus Pflanzen oder Pflanzenteilen umfaßt, die einen Gehalt an Catechinen oder Gallensäureestern von Catechinen aufweisen, wie beispielsweise den Blättern der Pflanzenfamilie Theaceae, insbesondere der Spezies Camellia sinensis (grüner Tee). Insbesondere vorteilhaft sind deren typische Inhaltsstoffe (wie z. B. Polyphenole bzw. Catechine, Coffein, Vitamine, Zucker, Mineralien, Aminosäuren, Lipide).

Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydroxyphenyl)-chroman-3,5,7-triol) darstellen. Auch Epicatechin ((2R,3R)-3,3',4',5,7-Flavanpentaol) ist ein vorteilhafter Wirkstoff im Sinne der vorliegenden Erfindung.

Vorteilhaft sind ferner pflanzliche Auszüge mit einem Gehalt an Catechinen, insbesondere Extrakte des grünen Tees, wie z. B. Extrakte aus Blättern der Pflanzen der Spezies Camellia spec., ganz besonders der Teesorten Camellia sinenis, C. assamica, C. taliensis bzw. C. irrawadiensis und Kreuzungen aus diesen mit beispielsweise Camellia japonica.

Bevorzugte Wirkstoffe sind ferner Polyphenole bzw. Catechine aus der Gruppe (-)-Catechin, (+)-Catechin, (-)-Catechingallat, (-)-Gallocatechingallat, (+)-Epicatechin, (-)-Epicatechin, (-)-Epicatechin Gallat, (-)-Epigallocatechin, (-)-Epigallocatechingallat.

Auch Flavon und seine Derivate (oft auch kollektiv "Flavone" genannt) sind vorteilhafte Wirkstoffe im Sinne der vorliegenden Erfindung. Sie sind durch folgende Grundstruktur gekennzeichnet (Substitutionspositionen angegeben):

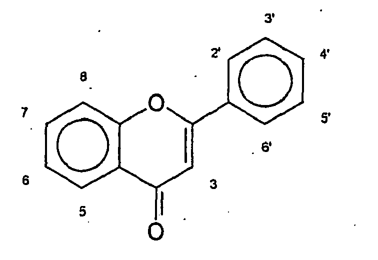

Einige der wichtigeren Flavone, welche auch bevorzugt in erfindungsgemäßen Zubereitungen eingesetzt werden können, sind in der nachstehenden Tabelle aufgeführt:

| | OH-Substitutionspositionen | | | | | | | |
|---|---|---|---|---|---|---|---|---|
| | 3 | 5 | 7 | 8 | 2' | 3' | 4' | 5' |
| Flavon | - | - | - | - | - | - | - | - |
| Flavonol | + | - | - | - | - | - | - | - |
| Chrysin | - | + | + | - | - | - | - | - |
| Galangin | + | + | + | - | - | - | - | - |
| Apigenin | - | + | + | - | - | - | + | - |
| Fisetin | + | - | + | - | - | + | + | - |
| Luteolin | - | + | + | - | - | + | + | - |
| Kämpferol | + | + | + | - | - | - | + | - |
| Quercetin | + | + | + | - | - | + | + | - |
| Morin | + | + | + | - | + | - | + | - |
| Robinetin | + | - | + | - | - | + | + | + |
| Gossypetin | + | + | + | + | - | + | + | - |
| Myricetin | + | + | + | - | - | + | + | + |

In der Natur kommen Flavone in der Regel in glycosidierter Form vor.

Erfindungsgemäß werden die Flavonoide bevorzugt gewählt aus der Gruppe der Substanzen der generischen Strukturformel 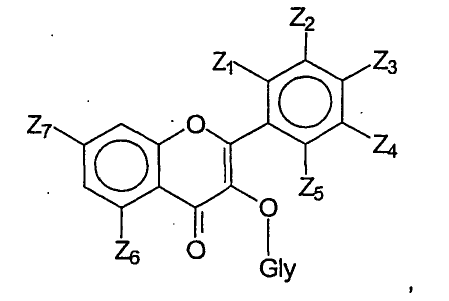 wobei Z₁ bis Z₇ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Erfindungsgemäß können die Flavonoide aber auch vorteilhaft gewählt werden aus der Gruppe der Substanzen der generischen Strukturformel 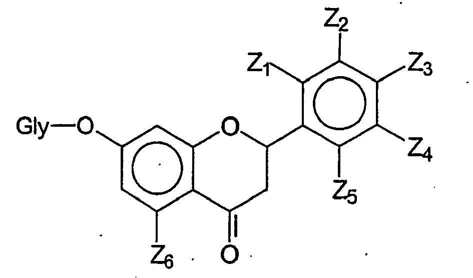 wobei Z₁ bis Z₆ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Bevorzugt können solche Strukturen gewählt werden aus der Gruppe der Substanzen der generischen Strukturformel 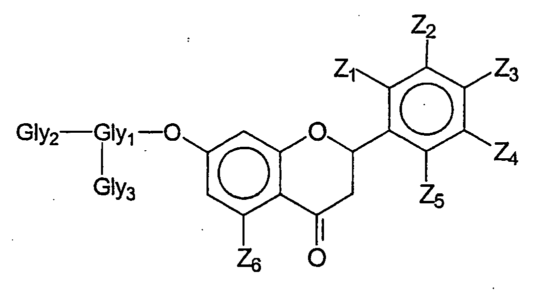 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Vorteilhaft werden Z₁ bis Z₅ unabhängig voneinander gewählt aus der Gruppe H, OH, Methoxy-, Ethoxy- sowie 2-Hydroxyethoxy-, und die Flavonglycoside haben die Struktur 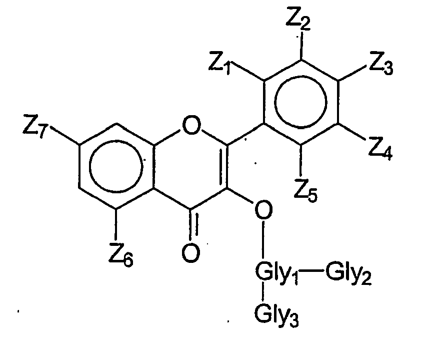 Besonders vorteilhaft werden die erfindungsgemäßen Flavonglycoside aus der Gruppe, welche durch die folgende Struktur wiedergegeben werden: 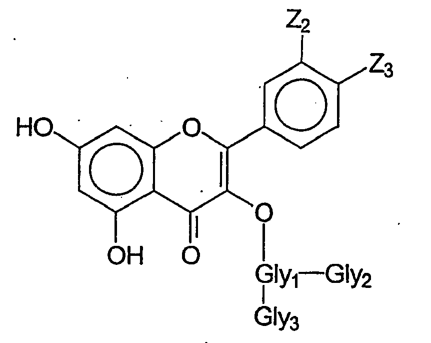 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Besonders vorteilhaft im Sinne der vorliegenden Erfindung ist, das oder die Flavonglycoside zu wählen aus der Gruppe α-Glucosylrutin, α-Glucosylmyricetin, α-Glucosylisoquercitrin, α-Glucosylisoquercetin und α-Glucosylquercitrin.

Erfindungsgemäß besonders bevorzugt ist α-Glucosylrutin.

Erfindungsgemäß vorteilhaft sind auch Naringin (Aurantiin, Naringenin-7-rhamnoglucosid), Hesperidin (3',5,7-Trihydroxy-4'-methoxyflavanon-7-rutinosid, Hesperidosid, Hesperetin-7-O-rutinosid). Rutin (3,3',4',5,7-Pentahydroxyflyvon-3-rutinosid, Quercetin-3-rutinosid, Sophorin, Birutan, Rutabion, Taurutin, Phytomelin, Melin), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Dihydrorobinetin (3,3',4',5',7-Pentahydroxyflavanon), Taxifolin (3,3',4',5,7-Pentahydroxyflavanon), Eriodictyol-7-glucosid (3',4',5,7-Tetrahydroxyflavanon-7-glucosid), Flavanomareïn (3',4',7,8-Tetrahydroxyflavanon-7-glucosid) und Isoquercetin (3,3',4',5,7-Pentahydroxyflavanon-3-(β-D-Glucopyranosid).

Vorteilhaft ist es auch, dem oder die Wirkstoffe aus der Gruppe der Ubichinone und Plastochinone zu wählen.

Ubichinone zeichnen sich durch die Strukturformel 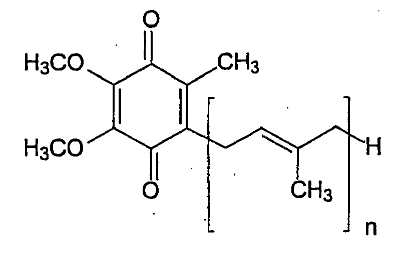 aus und stellen die am weitesten verbreiteten und damit am besten untersuchten Biochinone dar. Ubichinone werden je nach Zahl der in der Seitenkette verknüpften Isopren-Einheiten als Q-1, Q-2, Q-3 usw. oder nach Anzahl der C-Atome als U-5, U-10, U-15 usw. bezeichnet. Sie treten bevorzugt mit bestimmten Kettenlängen auf, z. B. in einigen Mikroorganismen und Hefen mit n=6. Bei den meisten Säugetieren einschließlich des Menschen überwiegt Q10.

Besonders vorteilhaft ist Coenzym Q10, welches durch folgende Strukturformel gekennzeichnet ist:

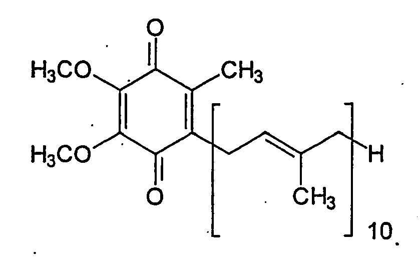

Plastochinone weisen die allgemeine Strukturformel 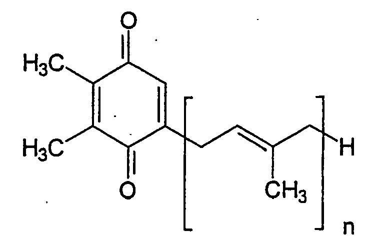 auf. Plastoschinone unterscheiden sich in der Anzahl n der Isopren-Reste und werden endsprechend bezeichnet, z. B. PQ-9 (n=9). Ferner existieren andere Plastochinone mit unterschiedlichen Substituenten am Chinon-Ring.

Auch Kreatin und/oder Kreatinderivate sind bevorzugte Wirkstoffe im Sinne der vorliegenden Erfindung. Kreatin zeichnet sich durch folgende Struktur aus:

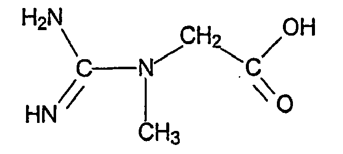

Bevorzugte Derivate sind Kreatinphosphat sowie Kreatinsulfat, Kreatinacetat, Kreatinascorbat und die an der Carboxylgruppe mit mono- oder polyfunktionalen Alkoholen veresterten Derivate.

Ein weiterer vorteilhafter Wirkstoff ist L-Carnitin [3-Hydroxy-4-(trimethylammonio)-buttersäurebetain]. Auch Acyl-Carnitine, welche gewählt aus der Gruppe der Substanzen der folgenden allgemeinen Strukturformel 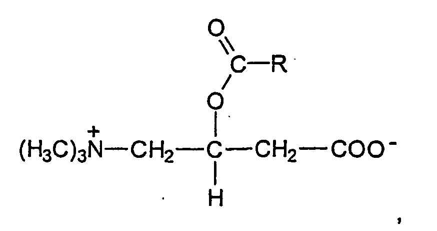 wobei R gewählt wird aus der Gruppe der verzweigten und unverzweigten Alkylreste mit bis zu 10 Kohlenstoffatomen sind vorteilhafte Wirkstoffe im Sinne der vorliegenden Erfindung. Bevorzugt sind Propionylcarnitin und insbesondere Acetylcarnitin. Beide Entantiomere (D- und L-Form) sind vorteilhaft im Sinne der vorliegenden Erfindung zu verwenden. Es kann auch von Vorteil sein, beliebige Enantiomerengemische, beispielsweise ein Racemat aus D- und L-Form, zu verwenden.

Weitere vorteilhafte Wirkstoffe sind Sericosid, Pyridoxol, Vitamin K, Biotin und Aromastoffe.

Die Liste der genannten Wirkstoffe bzw. Wirkstoffkombinationen, die in den erfindungsgemäßen Zubereitungen verwendet werden können, soll selbstverständlich nicht limitierend sein. Die Wirkstoffe können einzelnen oder in beliebigen Kombinationen miteinander verwendet werden.

Darüber hinaus eignen sich ausgewählte erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, insbesondere vorteilhaft zur Prophylaxe und Behandlung kosmetischer oder dermatologischer Hautveränderungen, wie sie z. B. bei der Hautalterung auftreten. Weiterhin vorteilhaft eignen sie sich gegen das Erscheinungsbild der trockenen bzw. rauhen Haut.

Hautalterung wird z. B. durch endogene, genetisch determinierte Faktoren verursacht. In Epidermis und Dermis kommt es alterungsbedingt z. B. zu folgenden Strukturschäden und Funktionsstörungen, die auch unter den Begriff "Senile Xerosis" fallen können:
a) Trockenheit, Rauhigkeit und Ausbildung von (Trockenheits-) Fältchen,
b) Juckreiz und
c) verminderte Rückfettung durch Talgdrüsen (z. B. nach dem Waschen).

Exogene Faktoren, wie UV-Licht und chemische Noxen, können kumulativ wirksam sein und z. B. die endogenen Alterungsprozesse beschleunigen bzw. sie ergänzen. In Epidermis und Dermis kommt es insbesondere durch exogene Faktoren z. B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z. B. Altersflecken) und
g) vergrößerte Anfälligkeit gegenüber mechanischem Stress (z. B. Rissigkeit).

In einer besonderen Ausführungsform betrifft die vorliegende Erfindung insbesondere Produkte zur Pflege der auf natürliche Weise gealterten Haut, sowie zur Behandlung der Folgeschäden der Lichtalterung, insbesondere der unter a) bis g) aufgeführten Phänomene.

Die erfindungsgemäßen kosmetischen und/oder dermatologischen Zubereitungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Eine weitere vorteilhafte Ausführungsform der vorliegenden Erfindung besteht in Sonnenschutz-Produkten.
Besonders vorteilhaft ist ein Zusatz von öllöslichen und/oder wasserlöslichen UV-Filtern und/oder UV-Strahlung absorbierender bzw. reflektierender anorganischer Pigmente.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescremes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂) Siliciums (SiO₂)_{,} Mangans (z. B. MnO), Aluminiums (Al₂O₃), Cers (z. B. Ce₂O₃), Mischoxide der entsprechenden Metalle sowie Abmischungen aus solchen Oxiden.

Solche Pigmente können im Sinne der vorliegenden Erfindung vorteilhaft oberflächlich behandelt ("gecoatet") sein, wobei beispielsweise ein amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophoben Schicht versehen werden.

Die Titandioxid- Pigmente können sowohl in der Kristallmodifikation Rutil als auch Anatas vorliegen und können im Sinne der vorliegenden Erfindung vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtung können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw.

Aluminiumoxidhydrat (auch: Alumina, CAS-Nr.: 1333-84-2), Natrium Hexametaphosphat (NaPO₃)₆, Natrium Metaphosphat (NaPO₃)ₙ, Siliziumdioxid (SiO₂) (auch: Silica, CAS-Nr.: 7631-86-9) oder Eisenoxid (Fe₂O₃). Diese anorganischen Oberflächenbeschichtungen können allein, in Kombination und/oder in Kombination mit organischen Beschichtungsmaterialien vorkommen.
Organische Oberflächenbeschichtungen im Sinne der vorliegenden Erfindung können bestehen aus pflanzlichem oder tierischem Aluminiumstearat, pflanzlicher oder tierischer Stearinsäure (Stearic acid), Laurinsäure (Lauric acid), Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure (Algic acid). Diese organischen Oberflächenbeschichtungen können allein, in Kombination und/oder in Kombination mit anorganischen Beschichtungsmaterialien vorkommen.

Beschriebene beschichtete und unbeschichtete Titandioxide können im Sinne vorliegender Erfindung auch in Form kommerziell erhältlicher öliger oder wäßriger Vordispersionen zur Anwendung kommen. Diesen Vordispersionen können vorteilhaft Dispergierhilfsmittel und/oder Lösungsvermittler (Solubilisationsvermittler) zugesetzt sein.

Geeignete Titandioxidpartikel und Vordispersionen von Titandioxidpartikeln im Sinne der vorliegenden Erfindung sind unter folgenden Handelsbezeichnungen bei den aufgeführten Firmen erhältlich:

| **Handelsname** | **Coating** / **Oberflächenüberzug** | **zusätzliche Bestandteile bei Vordispersionen** | **Hersteller** |
|---|---|---|---|
| MT-150W | None | - | Tayca Corporation |
| MT-150A | None | - | Tayca Corporation |
| MT-500B | None | - | Tayca Corporation |
| MT-600B | None | - | Tayca Corporation |
| MT-100TV | Aluminium Hydroxide Stearic acid | - | Tayca Corporation |
| MT-100Z | Aluminium Hydroxide Stearic acid | - | Tayca Corporation |
| MT-100T | Aluminium Hydroxide Stearic acid | - | Tayca Corporation |
| MT-500T | Aluminium Hydroxide Stearic acid | - | Tayca Corporation |
| MT-100S | Aluminium Hydroxide Lauric acid | - | Tayca Corporation |
| MT-100F | Stearic acid Iron oxide | - | Tayca Corporation |
| MT-100SA | Alumina Silica | - | Tayca Corporation |
| MT-500SA | Alumina Silica | - | Tayca Corporation |
| MT-600SA | Alumina Silica | - | Tayca Corporation |
| MT-100SAS | Alumina Silica Silicone | - | Tayca Corporation |
| MT-500SAS | Alumina Silica Silicone | - | Tayca Corporation |
| MT-500H | Alumina | - | Tayca Corporation |
| MT-100AQ | Silica Aluminiumhydroxide Alginic acid | - | Tayca Corporation |
| Eusolex T | Aqua Simethicone | - | Merck KgaA |
| Eusolex T-2000 | Alumina Simethicone | - | Merck KgaA |
| Eusolex T-Olio F | Silica Dimethylsilate | C₁₂₋₁₅ Alkylbenzoate | Merck KgaA |
| | Aqua | Calcium Polyhydroxystearate Silica Dimethylsilate | |
| Eusolex T-Olio P | Aqua Simethicone | Octyl Palmitate PEG-7 Hydrogenated Castor Oil Sorbitan Oleate Hydrogenated Castor Oil Beeswax Stearic acid | Merck KgaA |
| Eusolex T-Aqua | Aqua Alumina Sodium Metaphosphate | Phenoxyethanol Sodium Methylparaben Sodium Metaphosphate | Merck KgaA |
| Eusolex T-45D | Alumina Simethicone | Isononyl Isononanuate Polyglyceryl Ricinoleate | Merck KgaA |
| Kronos 1171 (Titandioxid 171) | None | - | Kronos |
| Titandioxid P25 | None | - | Degussa |
| Titandioxid T805 (Uvinul TiO₂) | Octyltrimethylsilan | - | Degussa |
| UV-Titan X610 | Alumina Dimethicone | - | Kemira |
| UV-Titan X170 | Alumina Dimethicone | - | Kemira |
| UV-Titan X161 | Alumina Silica Stearic Acid | - | Kemira |
| UV-Titan M210 | Alumina | - | Kemira |
| UV-Titan M212 | Alumina | Glycerol | Kemira |
| UV-Titan M262 | Alumina Silicone | - | Kemira |
| UV-Titan M160 | Alumina Silica Stearic Acid | - | Kemira |
| Tioveil AQ 10PG | Alumina Silica | Aqua Propylenglycol | Solaveil Uniquema |
| Mirasun TiW 60 | Alumina Silica | Aqua | Rhone-Poulenc |

Ganz besonders vorteilhafte Titandioxide sind Eusolex T-2000 und Eusolex T-aqua von der Fa. Merck, M-100 TV und MT-100 Z von der Fa. Tayca, Titandioxid T 805 von Degussa und Tioveil AQ 10PG von der Fa. Solaveil.

Eine weitere vorteilhafte Beschichtung der anorganische Pigmente besteht aus Dimethylpolysiloxan (auch: Dimethicon), einem Gemisch vollmethylierter, linearer Siloxanpolymere, die endständig mit Trimethylsiloxy-Einheiten blockiert sind.

Geeignete Zinkoxidpartikel und Vordispersionen von Zinkoxidpartikeln im Sinne der vorliegenden Erfindung sind unter folgenden Handelsbezeichnungen bei den aufgeführten Firmen erhältlich:

| **Handelsname** | **Hersteller** | **Coating** |
|---|---|---|
| Z- Cote HP1 | BASF | 2% Dimethicone |
| Z- Cote | BASF | / |
| ZnO NDM | H&R | 5% Dimethicone |
| ZnO Neutral | H&R | / |
| MZ- 300 | Tayca | / |
| MZ- 500 | Tayca | / |
| MZ- 700 | Tayca | / |
| MZ- 303S | Tayca | 3% Methicone |
| MZ- 505S | Tayca | 5% Methicone |
| MZ- 707S | Tayca | 7% Methicone |
| MZ- 303M | Tayca | 3% Dimethicone |
| MZ- 505M | Tayca | 5% Dimethicone |
| MZ- 707M | Tayca | 7% Dimethicone |
| Z- Sperse Ultra | Collaborative Laboratories | ZnO (>=56%)/ Dispersion in Dimethicone / Cyclomethicone / Ethylhexyl-Hydroxystearat-Benzoate |
| Samt- UFZO-450/D5 (60%) | Miyoshi Kasei | ZnO (60%)/ Dispersion in Cyclomethicone / Dimethicone |

Im Sinne der Erfindung sind die Zinkoxide Z-Cote und Z-Cote HP1 von der Fa. BASF, Zinkoxid NDM von der Fa. Haarmann & Reimer sowie MZ-505S von Tayca besonders bevorzugt.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches durch die chemische Strukturformel 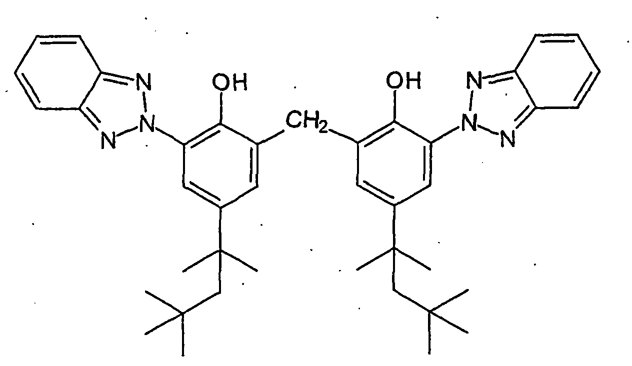 gekennzeichnet ist und unter der Handelsbezeichnung Tinosorb® M bei der CIBA-Chemikalien GmbH erhältlich ist.

Vorteilhaft enthalten erfindungsgemäße Zubereitungen Substanzen, die UV-Strahlung im UV-A- und/oder UV-B-Bereich absorbieren, wobei die Gesamtmenge der Filtersubstanzen z. B. 0,1 Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel für das Haar oder die Haut dienen.

Vorteilhafte weitere UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.

Eine weitere vorteilhafte UV-A-Filtersubstanzen im Sinne der vorliegenden Erfindung sind die Benzimidazol-Derivate, insbesondere das Phenylen-1,4-bis-(2-benzimidazyl)-3,3'-5,5'-tetrasulfonsäure, welches sich durch die folgende Struktur auszeichnet: 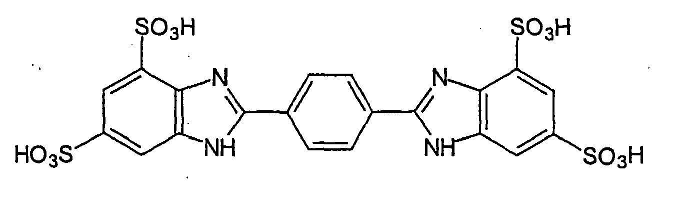 Vorteilhaft im Sinne der vorliegenden Erfindung sind ferner die entsprechenden Natrium-, Kalium- oder Triethanolammonium-Salze, insbesondere das Phenylen-1,4-bis-(2-benzimidazyl)-3,3'-5,5'-tetrasulfonsäure-bis-Natriumsalz 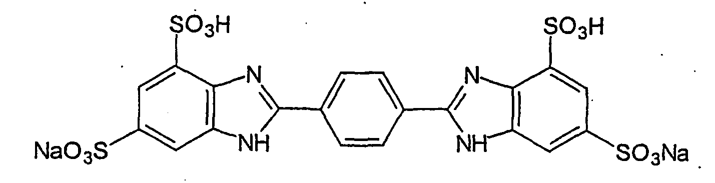 mit der INCI-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bomylidenmethyl)-Benzol und dessen Salze (besonders die entsprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure bezeichnet wird und sich durch die folgende Struktur auszeichnet: 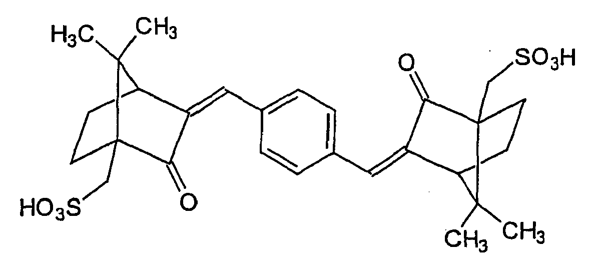 Benzol-1,4-di(2-oxo-3-bornylidenmethyl-10-sulfonsäure hat die INCI Bezeichnung Terephtalidene Dicampher Sulfonsäure (CAS.-Nr.: 90457-82-2) und ist beispielsweise unter dem Handelsnamen Mexoryl SX von der Fa. Chimex erhältlich.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur: 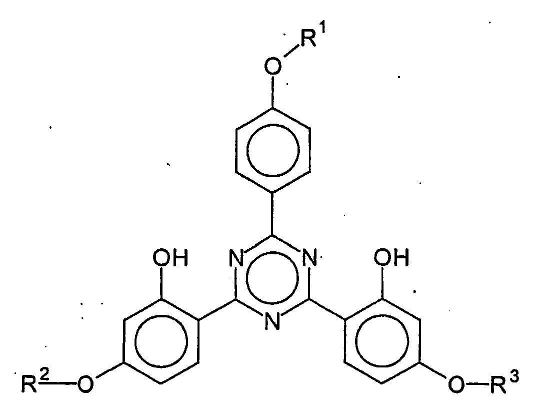 wobei R¹, R² und R³ unabhängig voneinander gewählt werden aus der Gruppe der verzweigten und unverzweigten Alkylgruppen mit 1 bis 10 Kohlenstoffatomen bzw. ein ein- .. zeines Wasserstoffatom darstellen. Insbesondere bevorzugt sind das 2,4-Bis-{[4-(2-Ethylhexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist.

Besonders vorteilhafte Zubereitungen im Sinne der vorliegenden Erfindung, die sich durch einen hohen bzw. sehr hohen UV-A-Schutz auszeichnen, enthalten neben der oder den erfindungsgemäßen Filtersubstanzen bevorzugt ferner weitere UV-A- und/oder Breitbandfilter, insbesondere Dibenzoylmethanderivate [beispielsweise das 4-(tert.-Butyl)-4'-methoxydibenzoylmethan], Phenylen-1,4-bis-(2-benzimidazyl)-3,3'-5,5'-tetrasulfonsäure und/oder ihre Salze, das 2,2'-Methylen-bis-(6-(2H-benzotriazol-2-yl)-4-(1,1,3,3-tetramethylbutyl)-phenol), das 1,4-di(2-oxo-10-Sulfo-3-bornylidenmethyl)-Benzol und/oder dessen Salze und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Auch andere UV-Filtersubstanzen, welche das Strukturmotiv 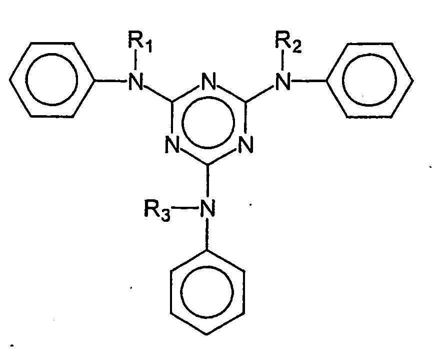 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 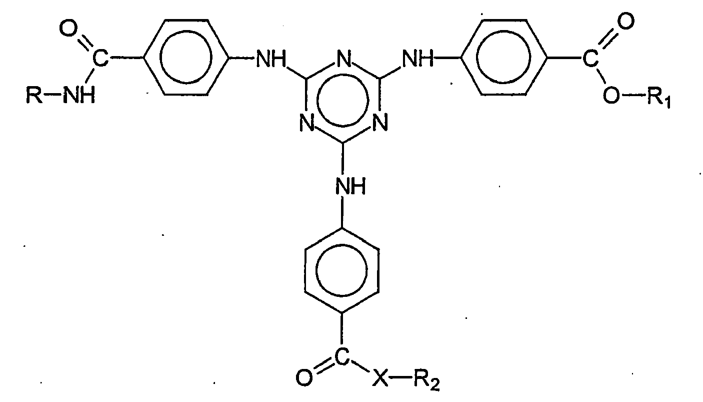 wiedergegeben wird, wobei
- R: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 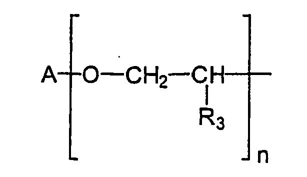 bedeutet, in welcher
- A: einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
- R₃: ein Wasserstoffatom oder eine Methylgruppe darstellt,
- n: eine Zahl von 1 bis 10 darstellt,
- R₂: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt, wenn X die NH-Gruppe darstellt, und
einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 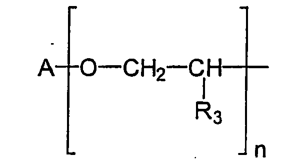 bedeutet, in welcher
- A: einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
- R₃: ein Wasserstoffatom oder eine Methylgruppe darstellt,
- n: eine Zahl von 1 bis 10 darstellt,
wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel 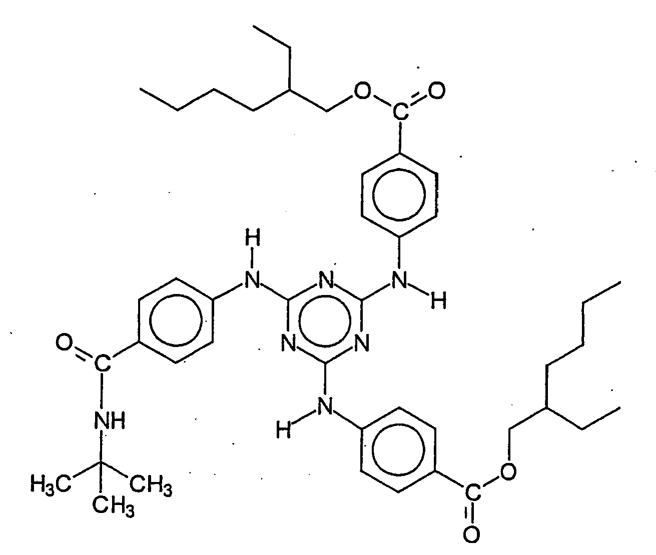 wiedergegeben wird, welches im Folgenden auch als Diethylhexylbutylamidotriazon (INCI: Diethylhexyl Butamidotriazone) bezeichnet wird und unter der Handelsbezeichnung UVASORB HEB bei Sigma 3V erhältlich ist.

Vorteilhaft im Sinne der vorliegenden Erfindung ist auch ein symmetrisch substituiertes s-Triazin, das 4,4',4"-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel 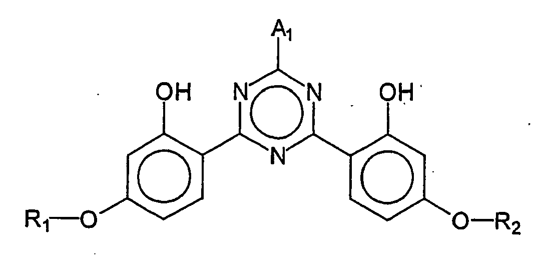 wiedergegeben wird, wobei R₁, R₂ und A₁ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxypropyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches durch die chemische Strukturformel 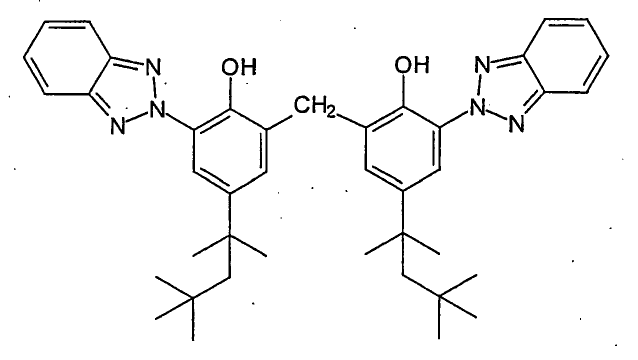 gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches durch die chemische Strukturformel 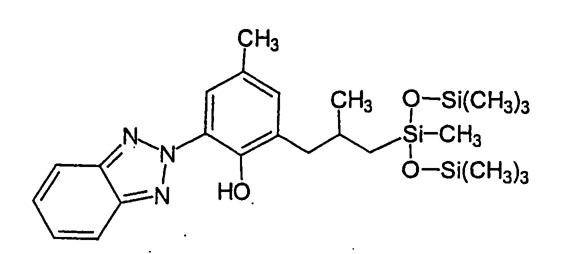 gekennzeichnet ist.

Die UV-B- und/oder Breitband-Filter können öllöslich oder wasserlöslich sein. Vorteilhafte öllösliche UV-B- und/oder Breitband-Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ 2,4,6-Trianilino-(p-carbo-2'-ethyl-1'-hexyloxy)-1,3,5-triazin;
■ Ester der Benzalmalonsäure, vorzugsweise 4-Methoxybenzalmalonsäuredi(2-ethylhexyl)ester;
■ Ester der Zimtsäure, vorzugsweise 4-Methoxyzimtsäure(2-ethylhexyl)ester, 4-Methoxyzimtsäureisopentylester,
■ Derivate des Benzophenons, vorzugweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.
■ 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol SLX® bei Hoffmann La Roche erhältlich ist.

Vorteilhafte wasserlösliche UV-B- und/oder Breitband-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Eine weitere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 erhältlich ist und sich durch folgende Struktur auszeichnet:

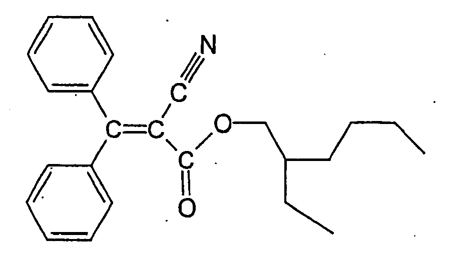

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-A-und/oder UV-B-Filter in kosmetische oder dermatologische Zubereitungen einzuarbeiten, beispielsweise bestimmte Salicylsäurederivate wie 4-Isopropylbenzylsalicylat, 2-Ethylhexylsalicylat (= Ethylhexylsalicylat), Homomenthylsalicylat.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.% bis 30 Gew.-%, vorzugsweise 0,5 bis 25 Gew.%, insbesondere 1,0 bis 20 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Ferner kann es gegebenenfalls von Vorteil sein, Filmbildner in die erfindungsgemäßen kosmetischen oder dermatologischen Zubereitungen einzuarbeiten, beispielsweise um die Wasserfestigkeit der Zubereitungen zu verbessern oder die UV-Schutzleistung zu erhöhen (UV-A- und/oder UV-B-Boosting). Geeignet sind sowohl wasserlösliche bzw. dispergierbare als auch fettlösliche Filmbildner, jeweils einzeln oder in Kombination miteinander.

Vorteilhafte wasserlöslich bzw. dispergierbare Filmbildner sind z. B. Polyurethane (z. B. die Avalure® -Typen von Goodrich), Dimethicone Copolyol Polyacrylate (Silsoft Surface® von der Witco Organo Silicones Group), PVP/VA (VA = Vinylacetat) Copolymer (Luviscol VA 64 Powder der BASF) etc.

Vorteilhafte fettlösliche Filmbildner sind z. B., die Filmbildner aus der Gruppe der Polymere auf Basis von Polyvinylpyrrolidon (PVP) 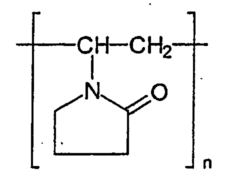 Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind, sowie das Tricontayl PVP und dergleichen mehr.

### Reinigungsmittel

Erfindungsgemäß können diese Emulsionen als kosmetische und dermatologische Zubereitungen auch als Reinigungsmittel eingesetzt werden.

Kosmetische Zubereitungen, die kosmetische Reinigungszubereitungen für die Haut darstellen, können in flüssiger oder fester Form vorliegen. Sie enthalten neben erfindungsgemäßen Wirkstoffkombinationen vorzugsweise mindestens eine anionische, nicht-ionische oder amphotere oberflächenaktive Substanz oder Gemische daraus, gewünschtenfalls einen oder mehrere Elektrolyten und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen.

### Repellentien- insektenabweisende Mittel

Eine weitere vorteilhafte Ausführungsform der vorliegenden Erfindung besteht in insektenabweisenden Mitteln.

Vorteilhafte Wirkstoffe für Repellents sind niedrig schmelzende oder flüssige Amide, Alkohole Ester und Ether mit Schmelzpunkten über 150°C, die bei Raumtemperatur nur langsam Verdampfen.

Als besonders vorteilhaft haben sich folgende Wirkstoffe einzeln oder in Kombination miteinander oder mit anderen erwiesen: 3-(N-n-Butyl-N-acetyl-amino)-propionsäureethylester (Handelname: Insekt-Repellent 3535 bei der Fa. Merck erhältlich), N,N-Diethyl-3-methylbenzamid (DEET), Dimethylphthalat, Ethylhexandiol, Caprylsäurediethylamid und natürliche Pflanzeöle wie Citronellöl, Eucalyptusöl, Lavendelöl, Nelkenöl.

### Selbstbräuner

Eine weitere vorteilhafte Ausführungsform der vorliegenden Erfindung besteht in Selbstbräunern.

Vorteilhafte Wirkstoffe für Selbstbräuner sind natürliche oder synthetische Ketole oder Aldole.
Als vorteilhaft haben sich Dihydroxyaceton (DHA), Glycerolaldehyd, Erythrulose, Alloxan, Hydroxymethylglyoxal, γ-Dialdehyd, 6-Aldo-D-Fructose, Ninhydrin und meso-Weinsäuredialdehyd erwiesen.

Als besonders vorteilhaft habe sich Mischungen der o.g. Wirkstoffe untereinander oder mit Mucondialdehyd oder/und Naphthochinone wie z.B. 5-Hydroxy-1,4-naphthochinon (Juglon) und 2-Hydroxy-1,4-Naphthochinon

### Beispielrezepturen

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

**A: Sprühbare W/O - Sonnenschutzemulsionen:**

| **1.** | |
|---|---|
| Cetyl Dimethicon Copolyol | 2 |
| Polyglyceryl-2 Dipolyhydroxystearat | 2 |
| Polysorbat-65 | 1 |
| PEG-100 Stearat | 0,5 |
| Cetyl Phosphat | 1 |
| Cyclomethicon | 10 |
| Caprylyl Methicon | 5 |
| Tinosorb ® S | 2 |
| Ethylhexyl Triazon | 4 |
| Octocrylen | 5 |
| Ethylhexyl Salicylat | 5 |
| Phenylbenzmidazol Sulfonat | 4 |
| Titandioxid T 805 ® | 3 |
| Zinkoxid Neutral | 1 |
| C12-15 Alkyl Benzoat | 2 |
| Butylen Glycol Dicaprylat/Dicaprat | 5 |
| Dicaprylyl Carbonat | 3 |
| Dihexyl Carbonat | 5 |
| Shea Butter | 0,75 |
| PVP Hexadecen Copolymer | 0,5 |
| Silsoft Surface ® | 1,0 |
| Glycerin | 10 |
| Xanthan Gummi | 0,1 |
| Vitamin E Acetat | 1 |
| EDTA | 0,01 |
| Magnesiumsulfat | 0,3 |
| DMDM Hydantoin | 0,01 |
| Ethanol | 4 |
| Farbstoff | q.s. |
| Parfüm | q.s. |
| Wassser | ad. 100 |

| **2.** | |
|---|---|
| Laurylmethicon Copolyol | 3 |
| Polyglyceryl-3 Düsostearat | 2 |
| Polysorbat-20 | 2 |
| Cetearyl Sulfat | 0,7 |
| Dimethicon | 2 |
| Phenyl Trimethicon | 5 |
| Tinosorb ® S | 3 |
| 4-Methylbenzyliden Campher | 4 |
| Ethylhexyl Methoxycinnamat | 10 |
| Homosalat | 7 |
| Diethylhexyl Butamidotriazon | 2 |
| Dimethico-Diethylbenzalmalonat | 3 |
| MT-100 Z ® | 2 |
| Z-Cote HP1 | 3 |
| Dicaprylyl Ether | 6 |
| Butylen Glycol Dicaprylat/Dicaprat | 2 |
| Mineral Öl | 7 |
| PVP Hexadecen Copolymer | 1,0 |
| Glycerin | 7,5 |
| Vitamin E Acetat | 0,5 |
| Magnesiumsulfat | 0,7 |
| Konkaben LMB ® | 0,12 |
| Methylparaben | 0,3 |
| Phenoxyethanol | 0,5 |
| Farbstoff | q.s. |
| Parfüm | q.s. |
| Wassser | ad. 100 |

| **3.** | |
|---|---|
| Cetyl Dimethicon Copolyol | 1,5 |
| Laurylmethicon Copolyol | 0,7 |
| Polyglyceryl-2 Dipolyhydroxystearat | 1,0 |
| Polysorbat-65 | 1 |
| PEG-100 Stearat | 1 |
| Cyclomethicon | 15 |
| Neo Heliopan AP ® | 2 |
| Butyl Methoxydibenzoylmethan | 1 |
| Ethylhexyl Triazon | 2 |
| 4-Methylbenzyliden Campher | 4 - |
| Ethylhexyl Salicylat | 10 |
| Phenylbenzmidazol Sulfonat | 1,5 |
| C12-15 Alkyl Benzoat | 5 |
| Dicaprylyl Carbonat | 4 |
| Dihexyl Carbonat | 6 |
| Shea Butter | 3 |
| Silsoft Surface ® | 0,50 |
| Glycerin | 5 |
| Butylen Glycol | 5 |
| Xanthan Gummi | 0,3 |
| Natriumchlorid | 1,2 |
| Glycin Soja | 1,5 |
| Ethanol | 5 |
| Farbstoff | q.s. |
| Parfüm | q.s. |
| Wassser | ad. 100 |

| **4** | |
|---|---|
| Cetyl Dimethicon Copolyol | 1,5 |
| Polyglyceryl-2 Dipolyhydroxystearat | 2 |
| Polysorbat-20 | 1 |
| Cetearyl Sulfat | 0,5 |
| Cyclomethicon | 3. |
| Neo Heliopan AP ® | 0,5 |
| Butyl Methoxydibenzoylmethan | 1,5 |
| Tinosorb M ® | 2 |
| Ethylhexyl Salicylat | 8 |
| Dimethico-Diethylbenzalmalonat | 1 |
| Z-Cote HP1 | 1,5 |
| C12-15 Alkyl Benzoat | 7,5 |
| Dicaprylyl Carbonat | 10 |
| Glycerin | 7,5 |
| Vitamin E Acetat | 1,5 |
| Natriumchlorid | 0,6 |
| DMDM Hydantoin | 0,02 |
| Methylparaben | 0,4 |
| Farbstoff | q.s. |
| Parfüm | q.s. |
| Wassser | ad. 100 |

| **5** | |
|---|---|
| Cetyl Dimethicon Copolyol | 3 |
| Polyglyceryl-2 Dipolyhydroxystearat | 1 |
| Isostearyl Diglyceryl Succinat | 0,3 |
| Polysorbat-65 | 1,5 |
| Cetyl Phosphat | 0,7 |
| Cetearyl Sulfat | 1 |
| Dimethicon | 2 |
| Cyclomethicon | 15 |
| Tinosorb ® S | 4 |
| Ethylhexyl Methoxycinnamat | 10 |
| Octocrylen | 7,5 |
| Ethylhexyl Salicylat | 6,5 |
| Phenylbenzmidazol Sulfonat | 4 |
| MT-100 Z ® | 0,5 |
| Zinkoxid Neutral | 4 |
| Dicaprylyl Carbonat | 4 |
| Dihexyl Carbonat | 6 |
| Mineral Öl | 6 |
| PVP Hexadecen Copolymer | 0,4. |
| Butylen Glycol | 7 |
| α-Glucosylrutin | 0,15 |
| EDTA | 0,15 |
| Magnesiumsulfat | 1 |
| Konkaben LMB ® | 0,1 |
| Phenoxyethanol | 1 |
| Repellent 3535 ® | 10,0 |
| Ethanol | 1 |
| Farbstoff | q.s. |
| Parfüm | q.s. |
| Wassser | ad. 100 |

| **6** | |
|---|---|
| Cetyl Dimethicon Copolyol | 2,5 |
| Laurylmethicon Copolyol | 0,7 |
| Polyglyceryl-2 Dipolyhydroxystearat | 1,0 |
| Polysorbat-65 | 1 |
| PEG-100 Stearat | 1 |
| Cyclomethicon | 20 |
| Tinosorb ® S | 3 |
| Butyl Methoxydibenzoylmethan | 1,5 |
| Tinosorb M ® | 1 |
| 4-Methylbenzyliden Campher | 1 |
| Octocrylen | 4 |
| Ethylhexyl Salicylal | 8 |
| Homosalat | 2 |
| Diethylhexyl Butamidotriazon | 2 |
| Phenylbenzmidazol Sulfonat | 2 |
| Titandioxid T 805 ® | 5 |
| PVP Hexadecen Copolymer. | 0,7 |
| Butylen Glycol | 7,5 |
| α-Glucosylrutin | 0,5 |
| Magnesiumsulfat | 0,7 |
| DMDM Hydantoin | 0,01 |
| Glycin Soja | 0,5 |
| Farbstoff | q.s. |
| Parfüm | q.s. |
| Wassser | ad. 100 |

| **7** | |
|---|---|
| Cetyl Dimethicon Copolyol | 3 |
| Polyglyceryl-2 Dipolyhydroxystearat | 2 |
| Polysorbat-65 | 0,5 |
| PEG-100 Stearat | 0,5 |
| Cetyl Phosphat | 1 |
| Dimethicon | 5 |
| Cyclomethicon | 7 |
| Caprylyl Methicon | 6 |
| Neo Heliopan AP ® | 2,5 |
| Butyl Methoxydibenzoylmethan | 2 |
| Ethylhexyl Triazon | 2 |
| Octocrylen | 2,5 |
| Dimethico-Diethylbenzalmalonat | 2 |
| Dicaprylyl Carbonat | 5 |
| Dihexyl Carbonat | 5 |
| Mineral Öl | 15 |
| Shea Butter | 2 |
| Glycerin | 4 |
| Butylen Glycol | 5 |
| Vitamin E Acetat | 0,75 |
| Natriumchlorid | 0,75 |
| Phenoxyethanol | 1 |
| Glycin Soja | 1 |
| Farbstoff | q.s. |
| Parfüm | q.s. |
| Wassser | ad. 100 |

| **8** | |
|---|---|
| Cetyl Dimethicon Copolyol | 1,5 |
| Polyglyceryl-3 Diisostearat | 2 |
| Polysorbat-65 | 2 |
| Cetearyl Sulfat | 0,75 |
| Dimethicon | 5 |
| Cyclomethicon | 5 |
| Phenyl Trimethicon | 2 |
| Neo Heliopan AP ® | 1 |
| Tinosorb ® S | 2 |
| Ethylhexyl Triazon | 3 |
| Ethylhexyl Methoxycinnamat | 5 |
| Dicaprylyl Ether | 8 |
| Butylen Glycol Dicaprylat/Dicaprat | 8 |
| Dicaprylyl Carbonat | 3 |
| Glycerin | 6 |
| Butylen Glycol | 10 |
| Natriumchlorid | 1 |
| Methylparaben | 0,2 |
| Ethanol | 7 |
| Farbstoff | q.s. |
| Parfüm | q.s. |
| Wassser | ad. 100 |

### B. Sprühbare W/O - Emulsionen:

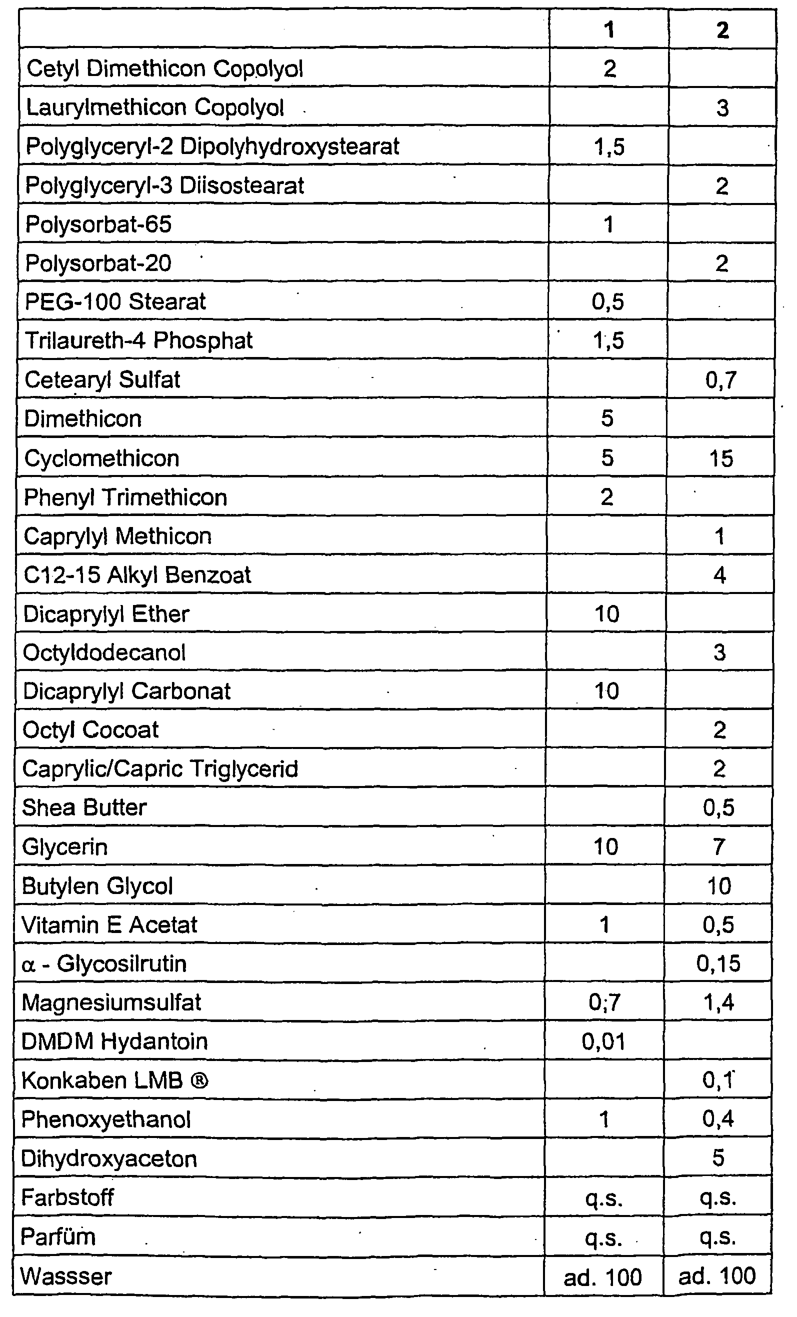

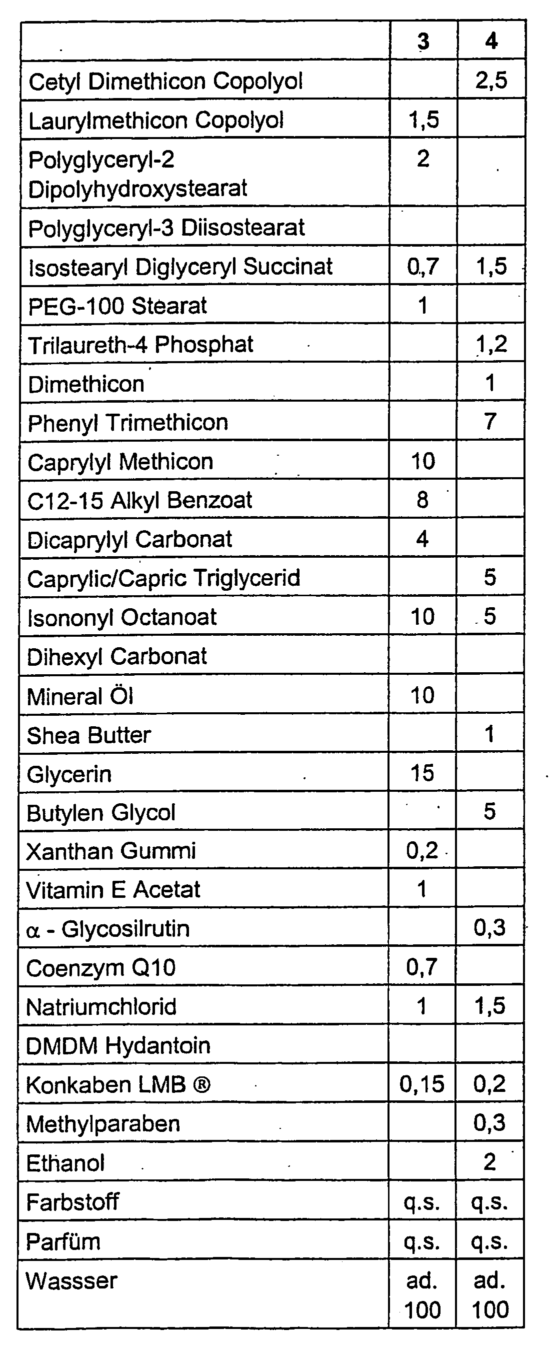

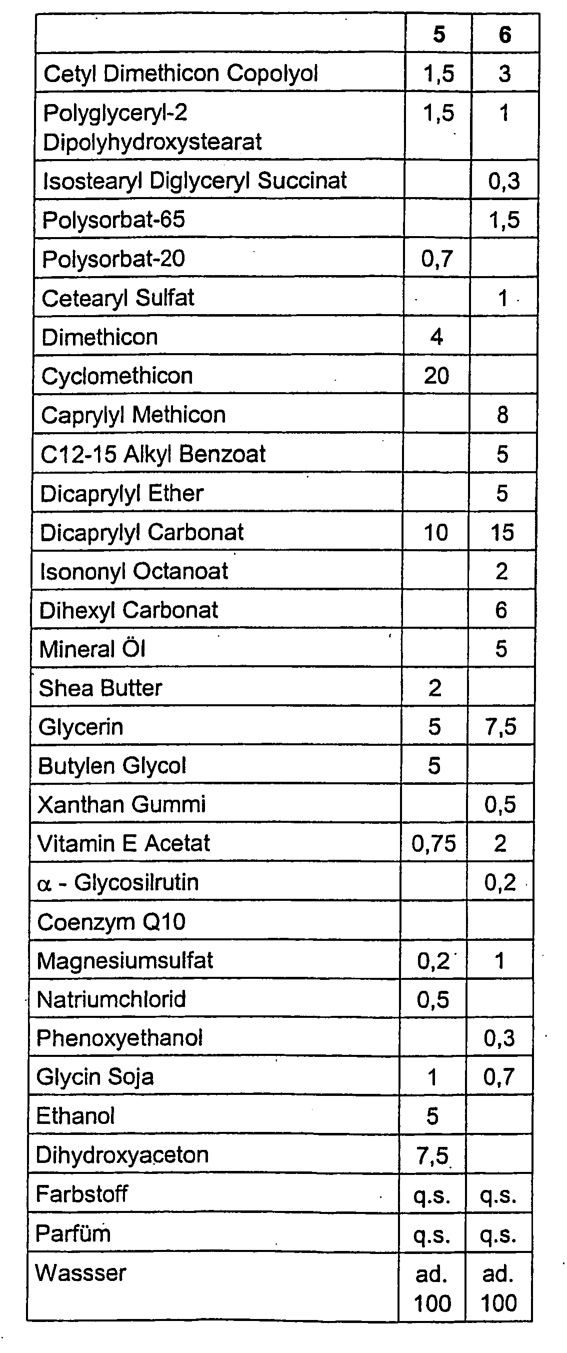

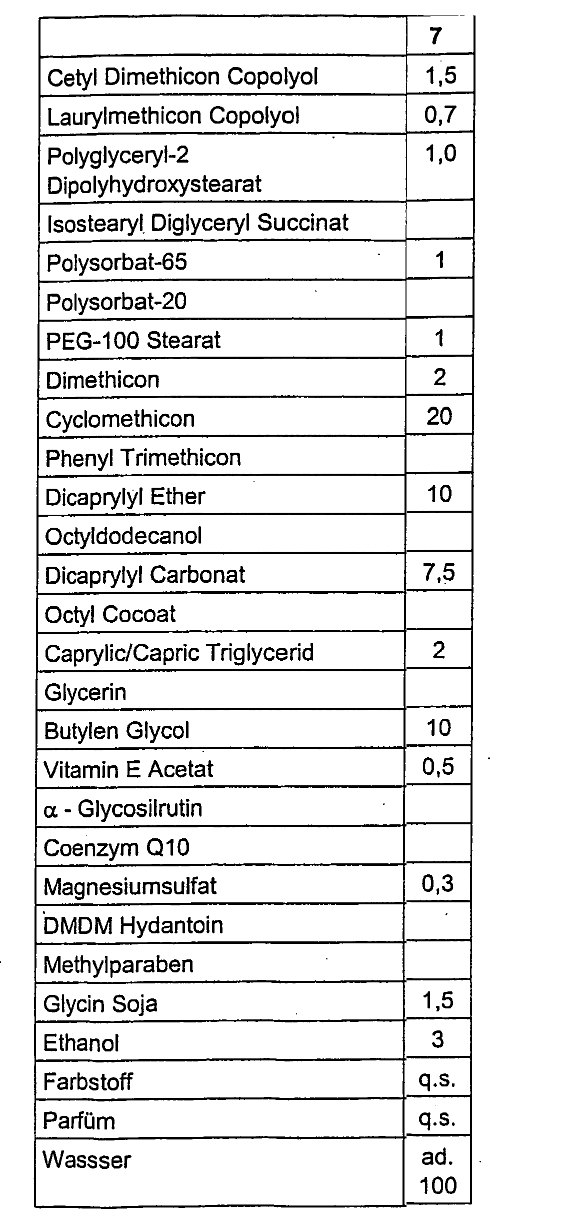

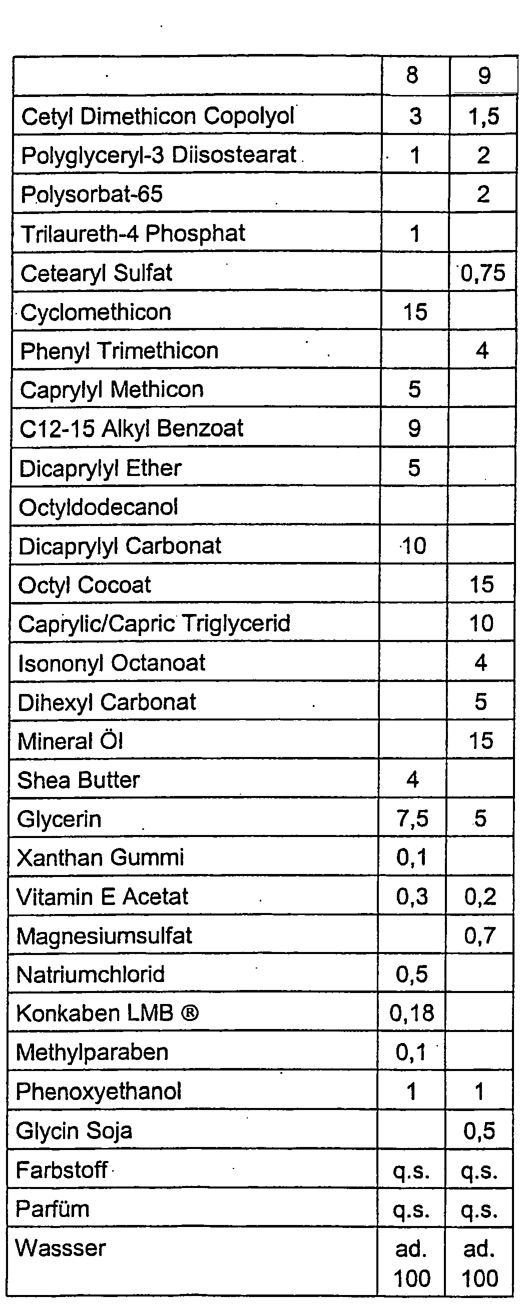

## Patentansprüche

1. Dünnflüssige, sprühbare W/O Emulsionen mit einem reduzierten Silikongehalt als kosmetische und dermatologische Zubereitungen, enthaltend - neben weiteren kosmetischen/dermatologischen Zusatz- bzw. Hilfsstoffen - ein Emulgatorsystem aus:
A mindestens einem Silikonemulgator (W/S) mit einem HLB-Wert ≤ 8.
B mindestens einem W/Q-Emulgator mit einem HLB-Wert < 7 und
C mindestens einem O/W-Emulgator mit einem HLB-Wert > 10,
und einem Silikonölgehalt unter 25 Gew.-% (bezogen auf das Gesamtgewicht der Zubereitung) und einer oder mehreren Lipide und/oder Öle enthaltenden Fettphase, **dadurch gekennzeichnet, dass**
der oder die W/O-Emulgator(en) B aus der Klasse der Polyglycerin-emulgatoren gewählt wird/werden.

2. Zubereitung nach Anspruch 1, **dadurch gekennzeichnet, dass** der oder die Silikonemulgator(en) A aus der Gruppe der Alkylmethiconcopolyole und/oder Alkyl-Dimethiconcolpolyole gewählt wird/werden.

3. Zubereitung nach mindestens einem der vorhergehenden Ansprüche, **dadurch gekennzeichnet, dass** der oder die O/W-Emulgator(en) C aus der Gruppe der ethoxylierten Polysorbate bzw. ethoxylierten Stearate, der Phophat- und/oder Sulfat- Emulgatoren gewählt wird/werden.

4. Zubereitung nach mindestens einem der vorhergehenden Ansprüche, **dadurch gekennzeichnet, dass** die Gesamtmenge der Emulgatoren A, B und C aus dem Bereich von 0,1 bis 15 Gew.-%, vorteilhaft von 0,5 bis 10 Gew.-%, insbesondere von 2 bis 14 Gew.-%, jeweils bezogen auf das Gesamtgewicht der Formulierung, gewählt ist.

5. Zubereitung nach mindestens einem der vorhergehenden Ansprüche, **dadurch gekennzeichnet, dass** die Fettphase oder ein Teil der Fettphase aus der Gruppe der cyclischen und/oder linearen Silikone und/oder deren Derivaten besteht und einen Gehalt von mindestens 2 Gew.% am Gesamtgewicht der Zubereitung ausmacht.

6. Zubereitung nach mindestens einem der vorhergehenden Ansprüche, **dadurch gekennzeichnet, dass** der Gehalt der an Silikonölen zwischen 2 und 25 Gew.-%, bezogen auf das Gesamtgewicht der Zubereitungen, bevorzugt 5 bis 20 Gew.%, insbesondere bevorzugt 10 bis 15 Gew.% beträgt.

7. Zubereitung nach mindestens einem der vorhergehenden Ansprüche, **dadurch gekennzeichnet, dass** die Fettphase oder ein Teil der Fettphase aus der Gruppe der polaren Öle und/oder Carbonsäureestem und/oder Dialkylether und/oder Dialkylcarbonate besteht.

8. Zubereitung nach mindestens einem der vorhergehenden Ansprüche, **dadurch gekennzeichnet, dass** der Gehalt der Fettphase zwischen 1 und 90 Gew.-%, bezogen auf das Gesamtgewicht der Zubereitungen, bevorzugt 2,5 - 80 Gew.-%, insbesondere bevorzugt 5 - 70 Gew.-% beträgt.

9. Zubereitung nach einem oder mehreren der vorhergehenden Ansprüche **dadurch gekennzeichnet, dass** sie ein oder mehrere öllösliche und/oder wasserlösliche Lichtschutzfilter, insbesondere UV-Filter aus der Gruppe der Triazine - ganz besonders 2,4-Bis-{[4-(2-Ethyl-hexyloxy)-2-hydroxy]-phenyl}-6-(4-methoxyphenyl)-1,3,5-triazin, Dioctylbutylamidotriazon, 4,4',4"-(1,3,5-Triazin-2,4,6-triyltriimino)-tris-benzoësäure-tris(2-ethylhexylester), der sulfonierten UV-Filter- ganz besonders Phenylen-1,4-bis-(2-benzimidazyl)-3,3'-5,5'-tetrasulfonsäure-bis-natriumsalz,2-Phenylbenzimidazol-5-sulfonsäure, Terephtalidene Dicampher Sulfonsäure -, der bei Raumtemperatur flüssigen UV-Filter - ganz besonders 4-Methoxyzimtsäure(2-ethylhexyl)ester, 2-Ethylhexyl-2-cyano-3,3-diphenylacrylat,2-Ethylhexyl-2-hydroxybenzoat, Homomenthylsalicylat -, der anorganische Pigmente - ganz besonders TiO2, ZnO - und der Benzotriazole - ganz besonders 2,2'-Methylen-bis-(6-(2H-benzotriazol-2-yl)-4-(1,1,3,3-tetramethylbutyl)-phenol), 2-(2H-benzotriazol-2-yl)-4-methyl-6-[2-methyl-3-[1,3,3,3-tetramethyl-1-[(trimethylsilyl)oxy]disiloxanyl]-propyl]-phenol - enthält.

10. Zubereitung nach einem oder mehreren der vorhergehenden Ansprüche **dadurch gekennzeichnet**, das sie ein oder mehrere zusätzliche Zusatz- und/oder Wirkstoffe, insbesondere Repellentien und/oder Selbstbräuner und/oder Pigmente, enthält.

11. Zubereitung nach einem oder mehreren der vorhergehenden Ansprüche **dadurch gekennzeichnet**, das sie eine hohe Wasserfestigkeit aufweist.

12. Zubereitung nach einem oder mehreren der vorhergehenden Ansprüche, die als Wirkstoff Vitamin E und/oder dessen Derivate enthalten.

13. Zubereitung nach einem oder mehreren der vorhergehenden Ansprüche, die als Wirkstoff α-Glucosylrutin und/oder dessen Derivate enthalten.

## Claims

1. Low-viscosity, sprayable W/O emulsions with a reduced silicone content as cosmetic and dermatological preparations, comprising - besides further cosmetic/dermatological additives and/or auxiliaries - an emulsifier system of:
A at least one silicone emulsifier (W/S) with an HLB value ≤ 8,
B at least one W/O emulsifier with an HLB value < and
C at least one O/W emulsifier with an HLB value > 10,
and a silicone oil content below 25% by weight (based on the total weight of the preparation) and one or more fatty phases comprising lipids and/or oils, **characterized in that**
the W/O emulsifier(s) B is/are selected from the class of polyglycerol emulsifiers.

2. Preparation according to Claim 1, **characterized in that** the silicone emulsifier(s) A is/are selected from the group of alkylmethicone copolyols and/or alkyldimethicone copolyols.

3. Preparation according to at least one of the preceding claims, **characterized in that** the O/W emulsifier(s) C is/are selected from the group of ethoxylated polysorbates and/or ethoxylated stearates, the phosphate and/or sulphate emulsifiers.

4. Preparation according to at least one of the preceding claims, **characterized in that** the total amount of the emulsifiers A, B and C is selected from the range from 0.1 to 15% by weight, advantageously from 0.5 to 10% by weight, in particular from 2 to 10% by weight, in each case based on the total weight of the formulation.

5. Preparation according to at least one of the preceding claims, **characterized in that** the fatty phase or part of the fatty phase consists of the group of cyclic and/or linear silicones and/or derivatives thereof and constitutes a content of at least 2% by weight of the total weight of the preparation.

6. Preparation according to at least one of the preceding claims, **characterized in that** the content of silicone oils is between 2 and 25% by weight, based on the total weight of the preparations, preferably 5 to 20% by weight, particularly preferably 10 to 15% by weight.

7. Preparation according to at least one of the preceding claims, **characterized in that** the fatty phase or part of the fatty phase consists of the group of polar oils and/or carboxylic acid esters and/or dialkyl ethers and/or dialkyl carbonates.

8. Preparation according to at least one of the preceding claims, **characterized in that** the content of the fatty phase is between 1 and 90% by weight, based on the total weight of the preparations, preferably 2.5-80% by weight, particularly preferably 5-70% by weight.

9. Preparation according to one or more the preceding claims, **characterized in that** it comprises one or more oil-soluble and/or water-soluble photo-protective filters, in particular UV filters from the group of the triazines - very particularly 2,4-bis{[4-(2-ethylhexyloxy)-2-hydroxylphenyl}-6-(4-methoxyphenyl)-1,3,5-triazine, dioctylbutylamidotriazone, tris(2-ethylhexyl) 4,4',4"-(1,3,5-triazine-2,4,6-triyltriimino)trisbenzoate, the sulfonated UV filters - very particularly phenylene-1,4-bis(2-benzimidazyl)-3,3'-5,5'-tetra-sulfonic acid bis-sodium salt, 2-phenyl-benzimidazole-5-sulfonic acid, terephthalidine-dicamphorsulfonic acid, the UV filters that are liquid at room temperature - very particularly 2-ethylhexyl 4-methoxycinnamate, 2-ethylhexyl 2-cyano-3,3-diphenylacrylate, 2-ethylhexyl 2-hydroxybenzoate, homomethyl salicylate, the inorganic pigments - very particularly TiO₂, ZnO - and the benzotriazoles - very particularly 2,2'-methylenebis(6-(2H-benzotriazol-2-yl)-4-(1,1,3,3-tetramethylbutyl)phenol, 2-(2H-benzotriazol-2-yl)-4-methyl-6-[2-methyl-3-[1,3,3,3-tetramethyl-1-[(trimethylsilyl)oxy]disiloxanyl]-propyl]phenol.

10. Preparation according to one or more of the preceding claims, **characterized in that** it comprises one or more additional additives and/or active ingredients, in particular repellents and/or self-tanning agents and/or pigments.

11. Preparation according to one or more of the preceding claims, **characterized in that** it has a high water resistance.

12. Preparation according to one or more of the preceding claims which comprises vitamin and/or derivatives thereof as active ingredient.

13. Preparation according to one or more of the preceding claims which comprises α-glucosylrutin and/or derivatives thereof as active ingredient.

## Revendications

1. Émulsions E/H fluides, pulvérisables, ayant une teneur réduite en silicone, sous forme de préparations cosmétiques et dermatologiques, contenant - en plus d'autres additifs ou adjuvants cosmétiques/dermatologiques - un système d'émulsifiants à base de :
A au moins un émulsifiant de silicone (E/S) ayant un indice HLB (équilibre hydrophile/lipophile) ≤ 8,
B au moins un émulsifiant E/H ayant un indice HLB < 7 et
C au moins un émulsifiant H/E ayant un indice HLB > 10,
et une phase lipidique ayant une teneur en huile de silicone inférieure à 25 % en poids (par rapport au poids total de la préparation) et une contenant un ou plusieurs lipides et/ou une ou plusieurs huiles, **caractérisées en ce que** l'émulsifiant ou les émulsifiants E/H B est/sont choisi(s) dans la classe des émulsifiants polyglycérol.

2. Préparation selon la revendication 1,
**caractérisée en ce que** l'émulsifiant ou les émulsifiants de silicone (A) est/sont choisi(s) dans le groupe des alkylméthicone-copolyols et/ou des alkyldiméthicone-copolyols.

3. Préparation selon au moins l'une des revendications précédentes, **caractérisée en ce que** l'émulsifiant ou les émulsifiants H/E C est/sont choisi(s) dans le groupe des polysorbates éthoxylés ou des stéarates éthoxylés, des émulsifiants phosphate et/ou sulfate.

4. Préparation selon au moins l'une des revendications précédentes, **caractérisée en ce que** la quantité totale des émulsifiants A, B et C est choisie dans la plage de 0,1 à 15 % en poids, avantageusement de 0,5 à à 10 % en poids, en particulier de 2 à 10 % en poids, chaque fois par rapport ai poids total de la composition,

5. Préparation selon au moins l'une des revendications précédentes, **caractérisée en ce que** la phase lipidique ou une partie de la phase lipidique consiste en le groupe des silicones linéaires et/ou cycliques et/ou de leurs dérivés et constitue une teneur d'au moins 2 % en poids du poids total de la préparation.

6. Préparation selon au moins l'une des revendications précédentes, **caractérisée en ce que** la teneur en huiles de silicone est comprise entre 2 et 25 % en poids, de préférence de 5 à 20 % en poids, de façon particulièrement préférée de 10 à 15 % en poids, par rapport au poids total des préparations.

7. Préparation selon au moins l'une des revendications précédentes, **caractérisée en ce que** la phase lipidique ou une partie de la phase lipidique consiste en le groupe des huiles polaires et/ou des esters d'acides carboxylique et/ou des éthers dialkyliques et/ou des carbonates de dialkyle.

8. Préparation selon au moins l'une des revendications précédentes, **caractérisée en ce que** la teneur en la phase lipidique est comprise entre 1 est 90 % en poids, de préférence entre 2,5 et 80 % en poids, de façon particulièrement préférée entre 5 et 70 % en poids, par rapport au poids total des préparations.

9. Préparation selon une ou plusieurs des revendications précédentes, **caractérisée en ce qu'**elle contient un ou plusieurs filtres photoprotecteurs hydrosolubles et/ou liposolubles, en particulier des filtres UV choisis dans le groupe des triazines - tout particulièrement la 2,4-bis-{[4-(2-éthyl-hexyloxy)-2-hydroxy]-phényl}-6-(4-méthoxyphényl}-1,3,5-triazine, la dioctylbutylamidotriazone, le 4,4',4"-(1,3,5-triazine-2,4,6-triyltriimino)-tris-benzoate de tris(2-éthylhexyle), des filtres UV sulfonés tout particulièrement le sel disodique d'acide phénylène-1,4-bis-(2-benzimidazyl)-3,3'-5,5'-tétrasulfonique, l'acide 2-phénylbenzimidazol-5-sulfonique, l'acide téréphtalidène-dicamphosulfonique des filtres UV liquides à la température ambiante tout particulièrement le 4-méthoxycinnamate de 2-éthylhexyle, le 2-cyano-3,3-diphénylacrylate de 2-éthylhexyle, le 2-hydroxybenzoate de 2-éthylhexyle, le salicylate d'homomenthyle -, des pigments inorganiques - tout particulièrement TiO₂, ZnO - et des benzotriazoles - tout particulièrement le 2,2'-méthylène-bis(6-(2H-benzotriazol-2-yl)-4-(1,1,3,3-tétraméthylbutyl)-phénol), le 2-(2H-benzotriazol-2-yl)-4-méthyl-6-[2-méthyl-3-[1,3,3,3-tétraméthyl-1-[(trimethylsilyl)oxy]disiloxanyl]-propyl]-phénol.

10. Préparation selon une ou plusieurs des revendications précédentes, **caractérisée en ce qu'**elle contient un ou plusieurs additifs et/ou actifs supplémentaires, en particulier des insectifuges et/ou des autobronzants et/ou des pigments.

11. Préparation selon une ou plusieurs des revendications précédentes, **caractérisée en ce qu'**elle présente une grande résistance à l'eau.

12. Préparation selon une ou plusieurs des revendications précédentes, **caractérisée en ce qu'**elle contient en tant qu'actif de la vitamine E et/ou ses dérivés.

13. Préparation selon une ou plusieurs des revendications précédentes, **caractérisée en ce qu'**elle contient en tant qu'actif de l'α-glucosylrutine et/ou ses dérivés.
